# EUROPEAN PATENT APPLICATION

(11) **EP 3 643 700 A1**
(43) Date of publication of application: **29.04.2020**
(21) Application number: 18820895.3
(22) Date of filing: 28.05.2018
(51) Int. Cl.: C07C 251/08, C07F 5/00, C23C 16/18, C23C 16/30, H01L 21/285

(54) **METAL ALKOXIDE COMPOUND, THIN-FILM-FORMING RAW MATERIAL, AND METHOD FOR PRODUCING THIN FILM**

(30) Priority: 21.06.2017 JP 2017121413
(71) Applicant: Adeka Corporation, Tokyo 116-8554 (JP)
(72) Inventor: SAKURAI, Atsushi, Tokyo 116-8554 (JP); HATASE, Masako, Tokyo 116-8554 (JP); OKADA, Nana, Tokyo 116-8554 (JP); NISHIDA, Akihiro, Tokyo 116-8554 (JP)
(74) Representative: Kador & Partner PartG mbB
(86) International application number: PCT/JP2018/020352
(87) International publication number: WO 2018/235530

(57) **Abstract**

The present invention provides a metal alkoxide compound represented by the following general formula (1): 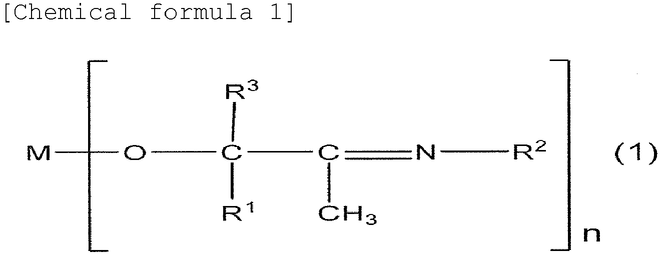 (in the formula, R¹ and R² each independently represent an alkyl group having 1 to 4 carbon atoms, R³ represents an alkyl group having 2 or 3 carbon atoms, M represents a scandium atom, an yttrium atom, a lanthanum atom, a cerium atom, a praseodymium atom, a neodymium atom, a promethium atom, a samarium atom, a europium atom, a gadolinium atom, a terbium atom, a dysprosium atom, a holmium atom, an erbium atom, a thulium atom, an ytterbium atom, or a lutetium atom, and n represents the valence represented by M.)

## Description

### Technical Field

The present invention relates to a metal alkoxide compound having a specific imino alcohol as a ligand, a thin-film-forming raw material containing the compound, and a method of producing a metal-containing thin film using the thin-film-forming raw material.

### Background Art

In elements of Group 3 in the periodic table, scandium (Sc), yttrium (Y), and lanthanoid from lanthanum (La) to lutetium (Lu) are collectively referred to as rare earth elements, and such rare earth elements are important elements in the field of electronics and optronics. Yttrium is a main constituent element of a Y-B-C-based superconductor. Lanthanum is a main constituent element of a ferroelectric PLZT. In addition, many lanthanoid elements are used as an additive for imparting functionality as a light-emitting material and the like.

Examples of a method of producing a thin film containing such an element include a sputtering method, an ion plating method, an MOD method such as a coating pyrolysis method and a sol-gel method, and a chemical vapor deposition method. However, a chemical vapor deposition (hereinafter simply referred to as "CVD") method including an atomic layer deposition (ALD) method is an optimal production process because it has many advantages such as excellent composition controllability and step coverage, being suitable for mass production, and enabling hybrid integration.

A large number of various raw materials are reported as metal supply sources used for chemical vapor deposition. However, for example, in Patent Document 1, a metal alkoxide compound that can be used as a thin-film-forming raw material is disclosed. In addition, in Patent Document 2, an alkoxide compound useful as a thin-film-forming raw material in the ALD method is disclosed, and a general formula representing a compound of the present invention is disclosed. However, Patent Document 2 does not specifically exemplify a metal alkoxide compound of the present invention and neither discloses nor specifically suggests favorable effects of the metal alkoxide compound of the present invention.

### Prior Art Documents

### Patent Documents

[Patent Document 1] WO 2014/077089
[Patent Document 2] Japanese Patent Laid-Opne No. 2015-205837

### Summary of the Invention

### Problems to be Solved by the Invention

When a raw material for chemical vapor deposition or the like is vaporized to form a metal-containing thin film on a surface of a substrate, a thin-film-forming material that can produce a metal-containing thin film having a high vapor pressure, a low melting point, and high quality is required. None of conventionally known thin-film-forming materials exhibits such a physical property. Among these, in order to improve productivity, a material having a low melting point is strongly required because it is necessary to improve transportability.

Therefore, an object of the present invention is to provide a metal alkoxide compound having a high vapor pressure and a melting point lower than that of a conventionally known compound, a thin-film-forming raw material containing the same, and a method of producing a thin film for forming a metal-containing thin film using the raw material.

### Means for Solving the Problem

As a result of repeated examinations, the inventors found that a specific metal alkoxide compound can address the above problem and thus developed the present invention.

That is, the present invention provides a metal alkoxide compound represented by the following general formula (1), a thin-film-forming raw material containing the same, and a method of producing a thin film for forming a metal-containing thin film using the raw material. 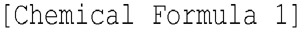 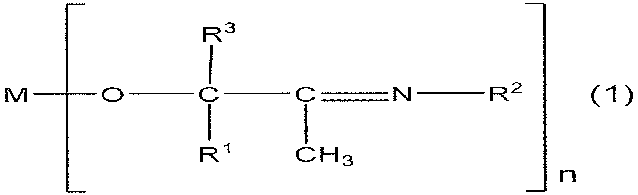 (in the formula, R¹ and R² each independently represent an alkyl group having 1 to 4 carbon atoms, R³ represents an alkyl group having 2 or 3 carbon atoms, M represents a scandium atom, an yttrium atom, a lanthanum atom, a cerium atom, a praseodymium atom, a neodymium atom, a promethium atom, a samarium atom, a europium atom, a gadolinium atom, a terbium atom, a dysprosium atom, a holmium atom, an erbium atom, a thulium atom, an ytterbium atom, or a lutetium atom, and n represents the valence represented by M.)

### Effects of the Invention

According to the present invention, it is possible to obtain a metal alkoxide compound having a high vapor pressure and a melting point lower than that of a conventionally known compound. The metal alkoxide compound of the present invention is particularly suitable as a thin-film-forming raw material for forming a metal thin film using a CVD method, and most especially, can be preferably used as a thin-film-forming raw material for forming a metal thin film using an ALD method.

### Brief Description of Drawings

Fig. 1 is a schematic view showing an example of a chemical vapor deposition device used in a method of producing a metal-containing thin film according to the present invention.
Fig. 2 is a schematic view showing another example of the chemical vapor deposition device used in a method of producing a metal-containing thin film according to the present invention.
Fig. 3 is a schematic view showing another example of the chemical vapor deposition device used in a method of producing a metal-containing thin film according to the present invention.
Fig. 4 is a schematic view showing another example of the chemical vapor deposition device used in a method of producing a metal-containing thin film according to the present invention.

### Best Mode for Carrying Out the Invention

A metal alkoxide compound of the present invention is represented by general formula (1) and is suitable as a precursor in a thin film production method including a vaporization process such as a CVD method and is a precursor that can be applied for an ALD method and thus particularly suitable as a precursor used in the ALD method.

In general formula (1), R¹ and R² each independently represent an alkyl group having 1 to 4 carbon atoms, R³ represents an alkyl group having 2 or 3 carbon atoms, M represents a scandium atom, an yttrium atom, a lanthanum atom, a cerium atom, a praseodymium atom, a neodymium atom, a promethium atom, a samarium atom, a europium atom, a gadolinium atom, a terbium atom, a dysprosium atom, a holmium atom, an erbium atom, a thulium atom, an ytterbium atom, or a lutetium atom, and n represents the valence represented by M.

In general formula (1), examples of alkyl groups having 1 to 4 carbon atoms represented by R¹ and R² include methyl, ethyl, propyl, isopropyl, butyl, isobutyl, sec-butyl, and tert-butyl groups. Examples of alkyl groups having 2 or 3 carbon atoms represented by R³ include ethyl, propyl and isopropyl groups, n represents the valence of the atom represented by M. It is known that a metal atom represented by M in general formula (1) has various valences, and among them, regarding a stable valence, for example, a case in which n is 2 when M is a europium atom or an ytterbium atom, a case in which n is 3 when M is a scandium atom, an yttrium atom, a lanthanum atom, a praseodymium atom, a neodymium atom, a promethium atom, a samarium atom, a gadolinium atom, a terbium atom, a dysprosium atom, a holmium atom, an erbium atom, a thulium atom, or a lutetium atom, and a case in which n is 4 when M is cerium may be exemplified.

The metal alkoxide compound represented by general formula (1) may have optical activity, but the metal alkoxide compound of the present invention may be any of an R form and an S form without particular distinction or a mixture containing an R form and an S form in a certain ratio. Here, the racemate is inexpensive to produce.

When a method of producing a thin film including a process of vaporizing the metal alkoxide compound represented by general formula (1) is used, a compound having a high vapor pressure and a low melting point is preferable. Therefore, regarding R¹ to R³ in general formula (1), specifically, R¹ is preferably a methyl or ethyl group, R² is preferably an ethyl group, and R³ is preferably an ethyl or propyl group. Among these, a metal alkoxide compound in which R¹ is a methyl or ethyl group and R² is an ethyl group is particularly preferable because it has strong effects of increasing a vapor pressure and reducing a melting point, and a metal alkoxide compound in which R¹ is an methyl or ethyl group, R² is an ethyl group, and R³ is an ethyl or propyl group is particularly preferable. In addition, when a metal alkoxide compound is used in a method of producing a thin film according to an MOD method not including a vaporization process, R¹ to R³ can be arbitrarily selected according to the solubility in a solvent to be used, a thin film formation reaction, and the like.

While the metal alkoxide compound of the present invention is represented by general formula (1), it is a concept including a case in which a ring structure in which a terminal donor group in the ligand is coordinated to a metal atom is formed, that is, a case in which it is represented by the following general formula (1-A) together without distinction. 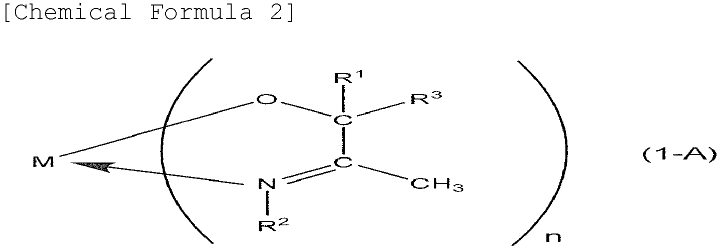 (In the formula, R¹ and R² each independently represent an alkyl group having 1 to 4 carbon atoms, R³ represents an alkyl group having 2 or 3 carbon atoms, M represents a scandium atom, an yttrium atom, a lanthanum atom, a cerium atom, a praseodymium atom, a neodymium atom, a promethium atom, a samarium atom, a europium atom, a gadolinium atom, a terbium atom, a dysprosium atom, a holmium atom, an erbium atom, a thulium atom, an ytterbium atom, or a lutetium atom, and n represents the valence represented by M.)

Specific examples of the metal alkoxide compound represented by general formula (I) include Compound No. 1 to Compound No. 357. Here, "Me" in the following chemical formula represents a methyl group, "Et" represents an ethyl group, "nPr" represents a propyl group, and "iPr" represents an isopropyl group.

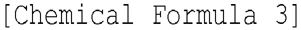

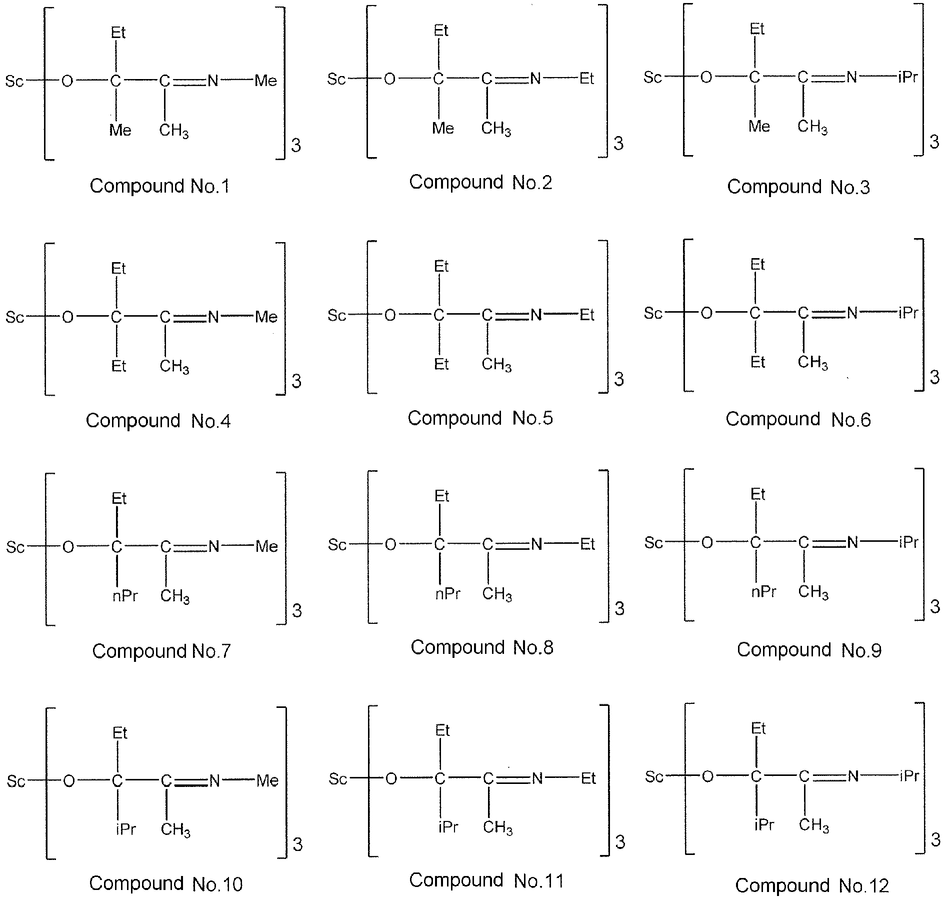

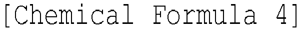

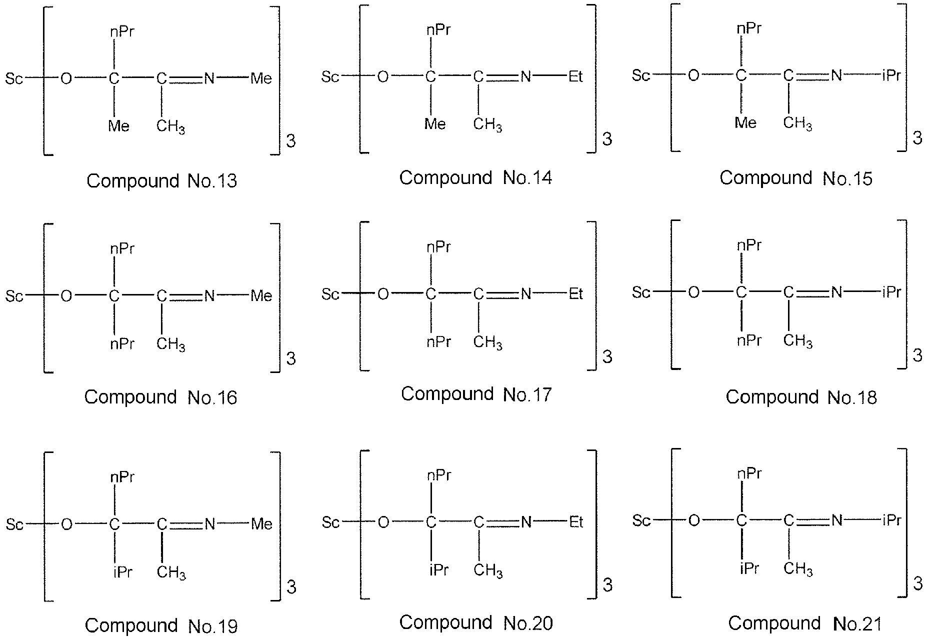

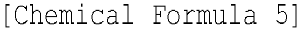

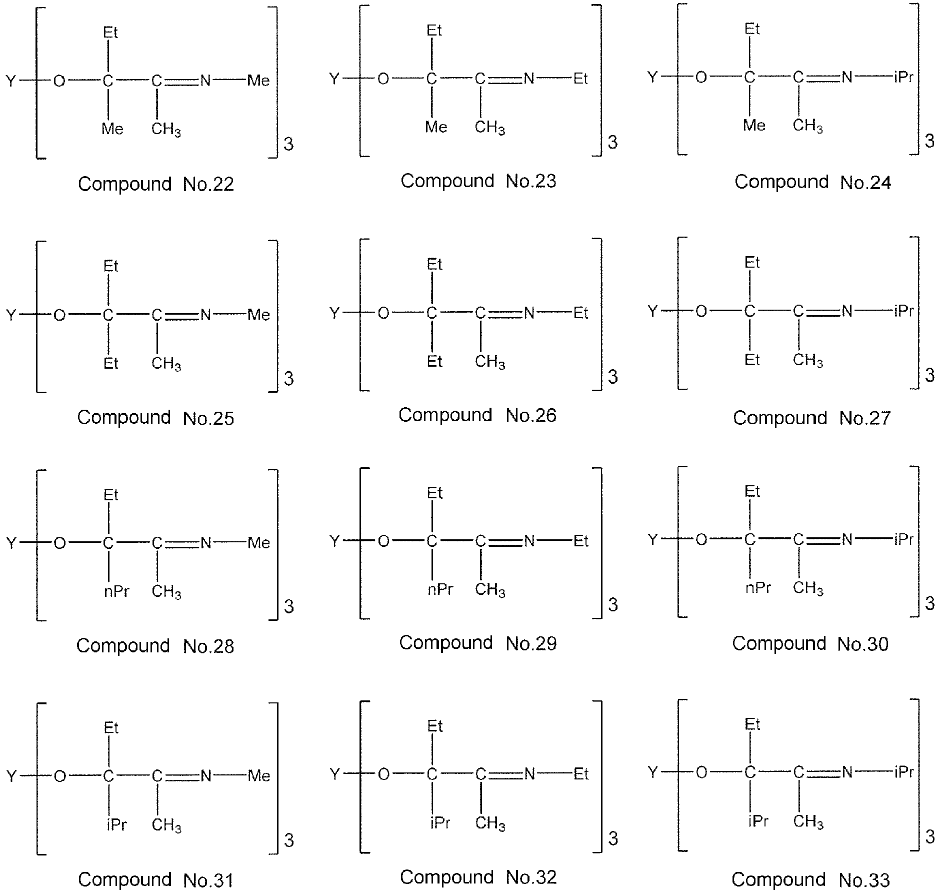

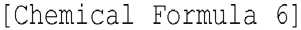

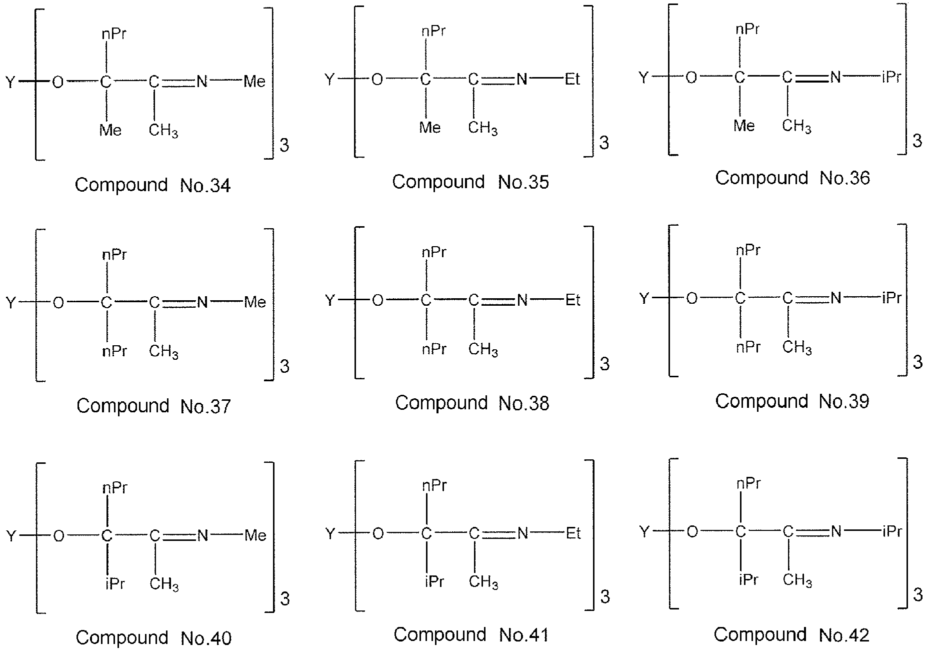

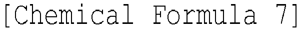

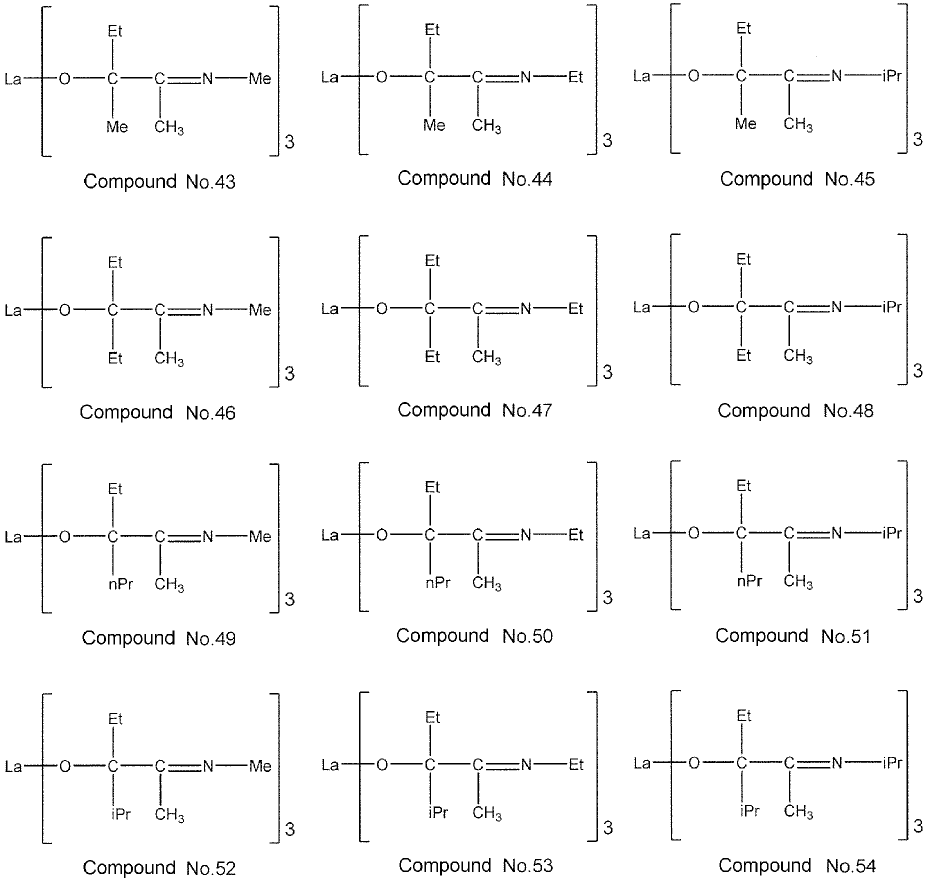

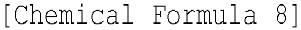

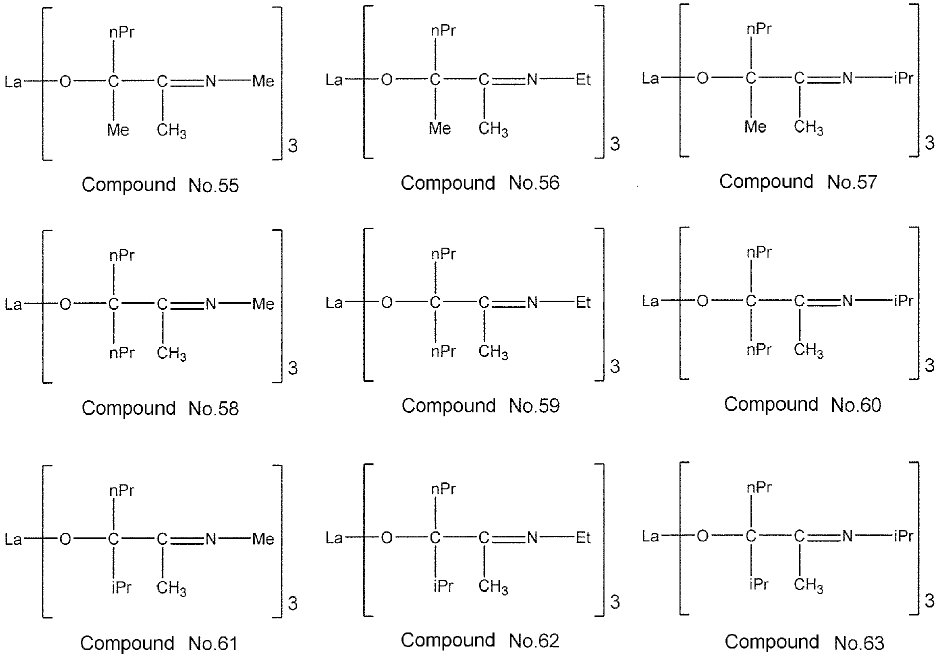

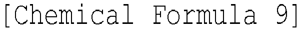

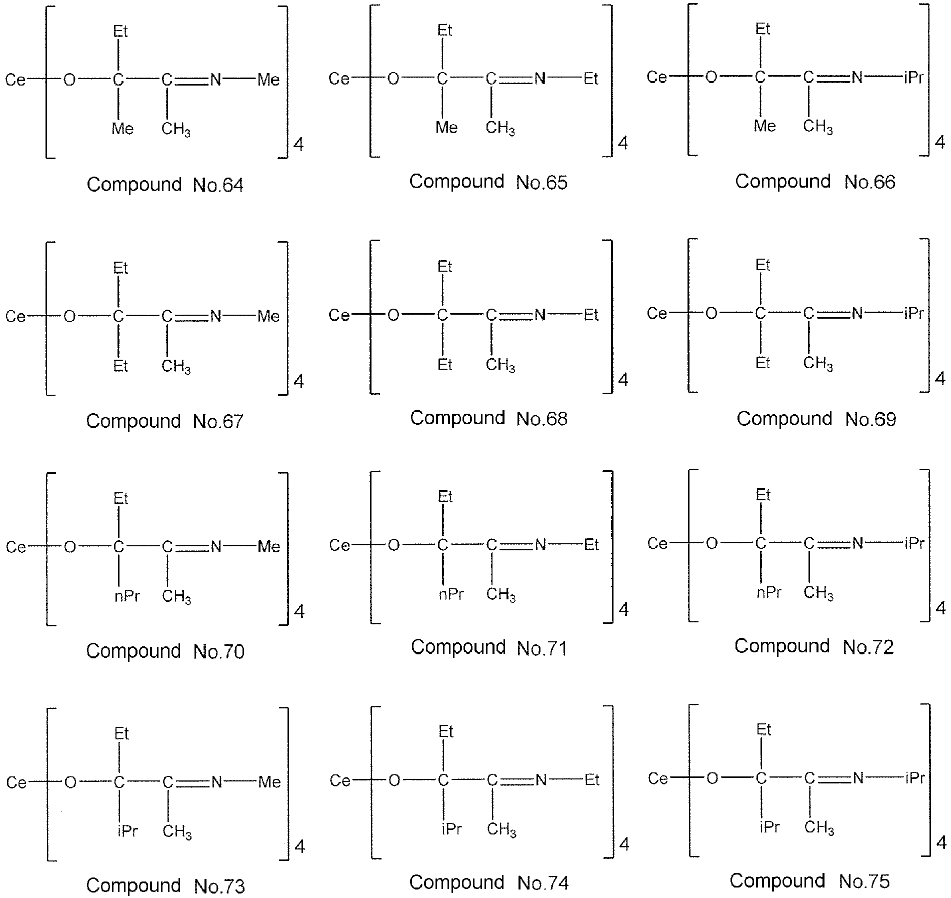

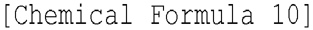

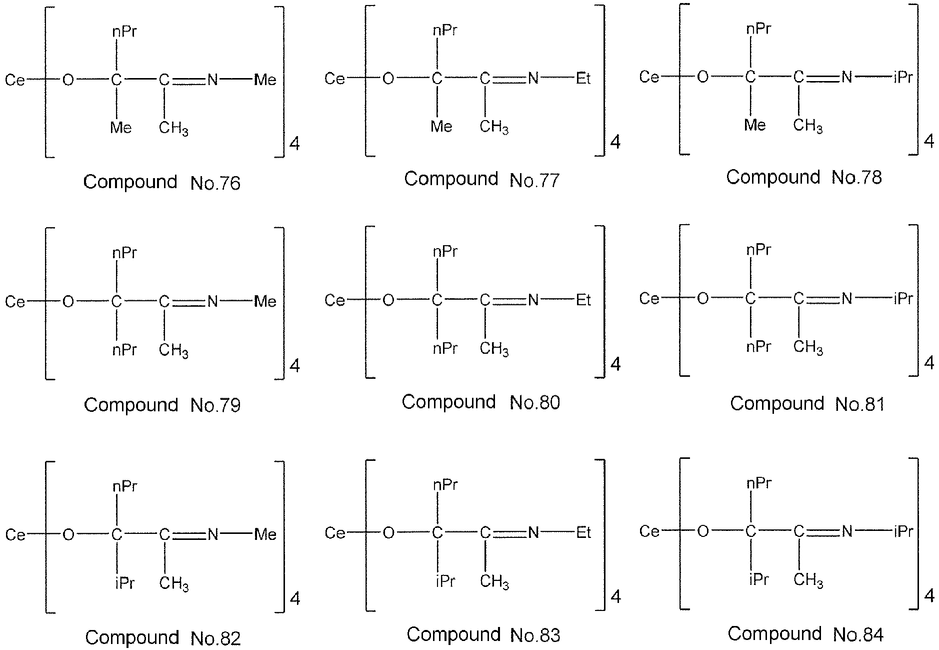

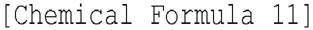

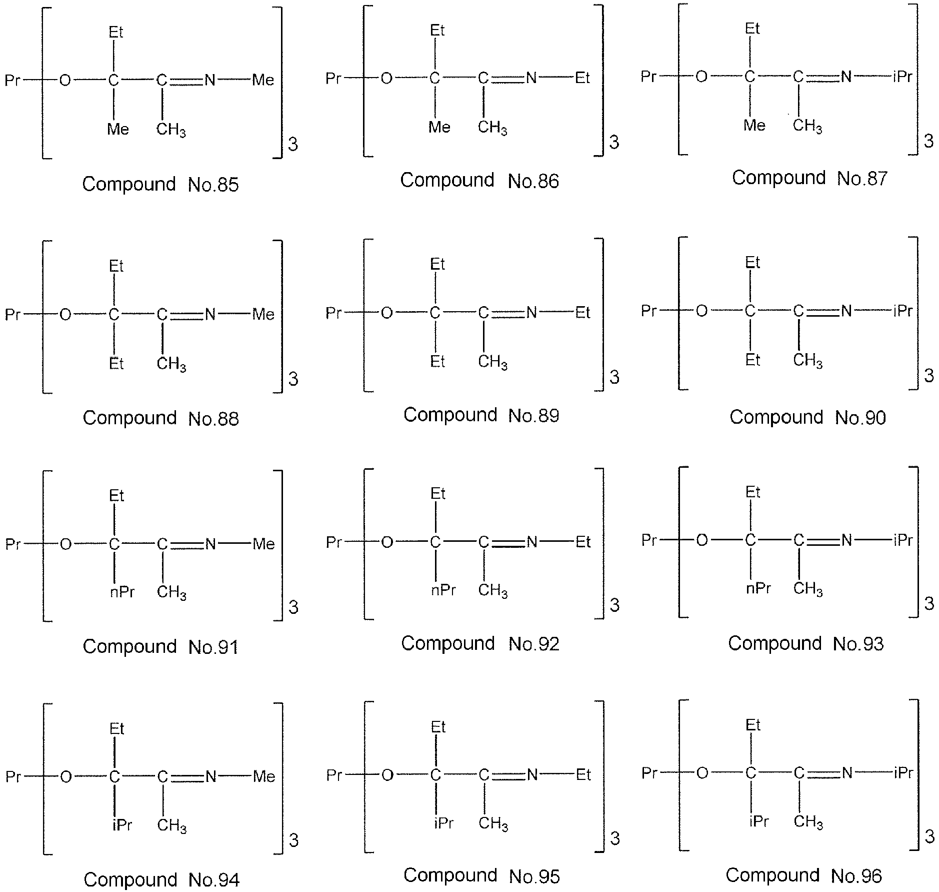

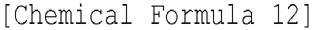

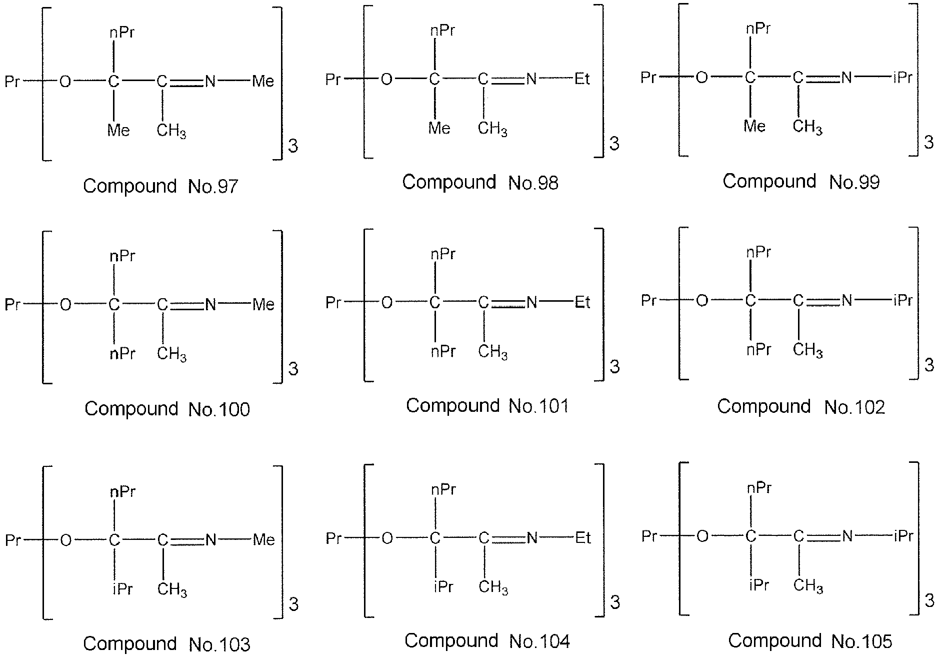

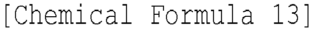

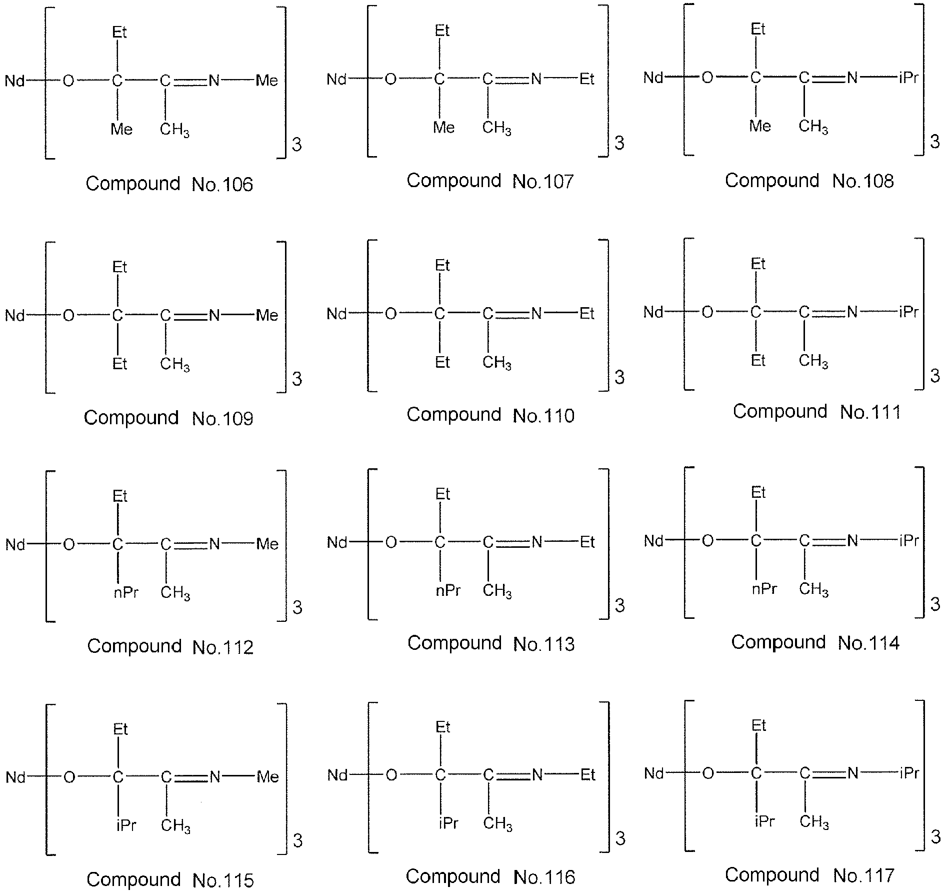

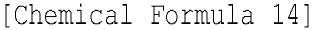

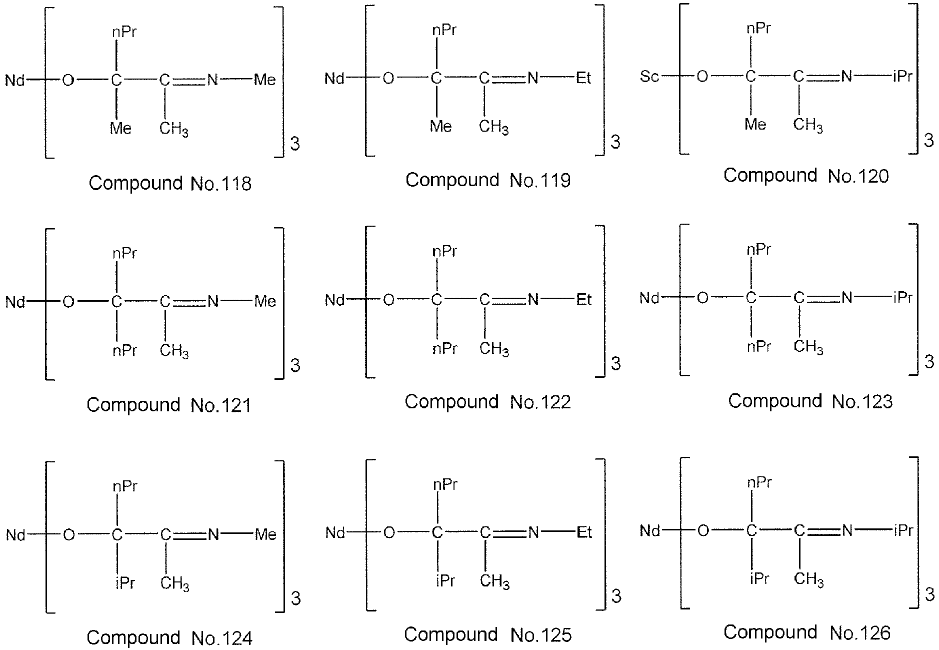

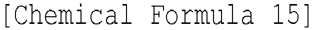

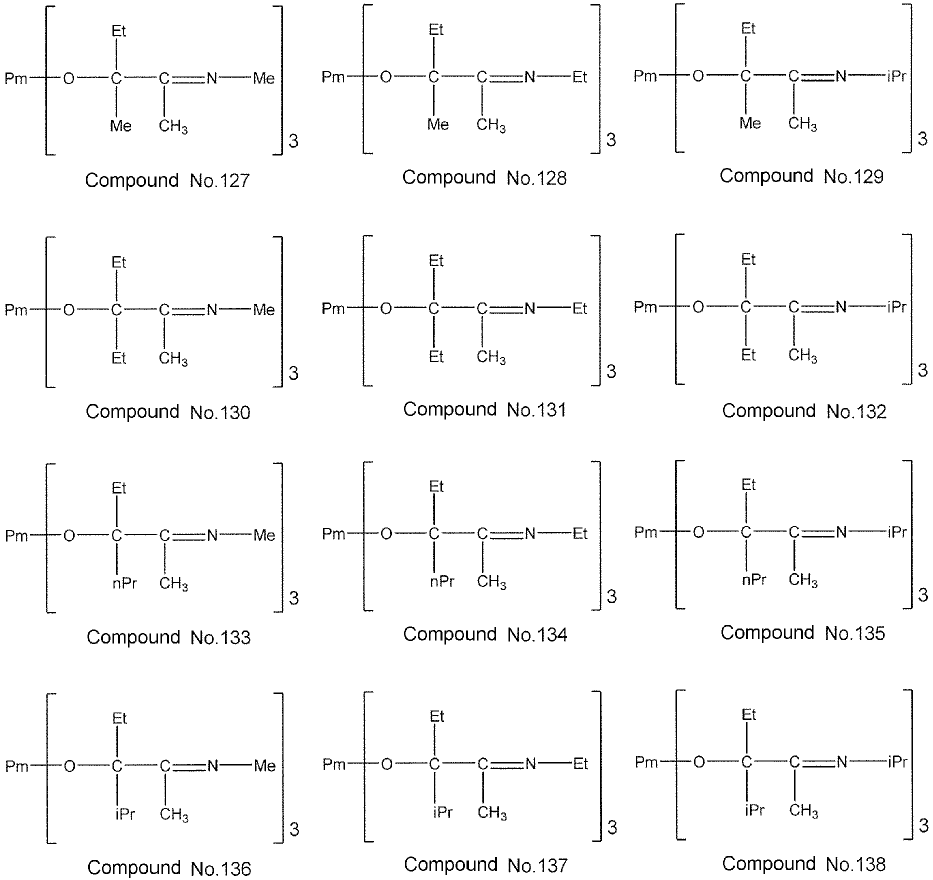

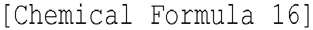

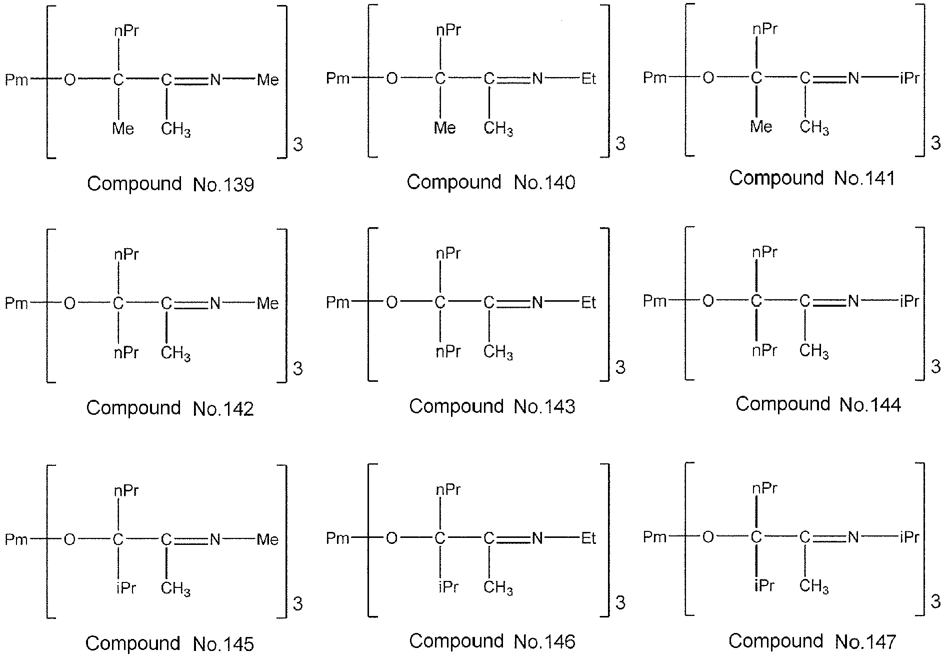

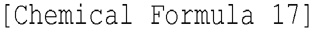

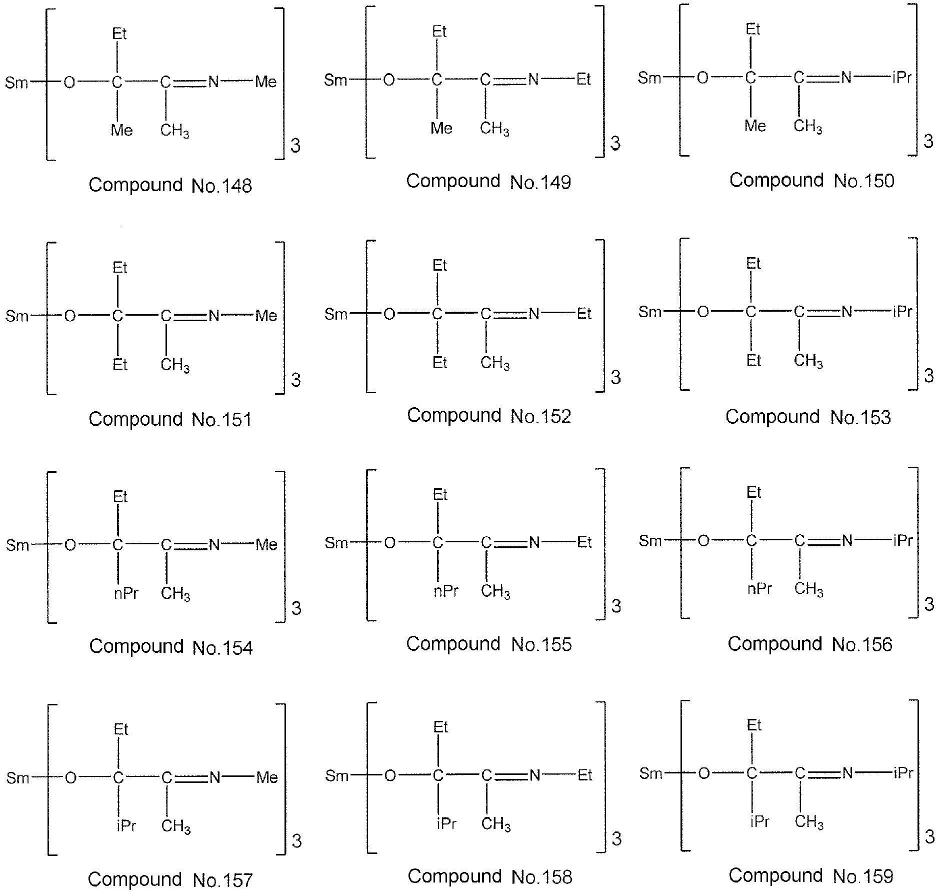

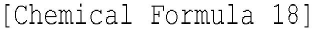

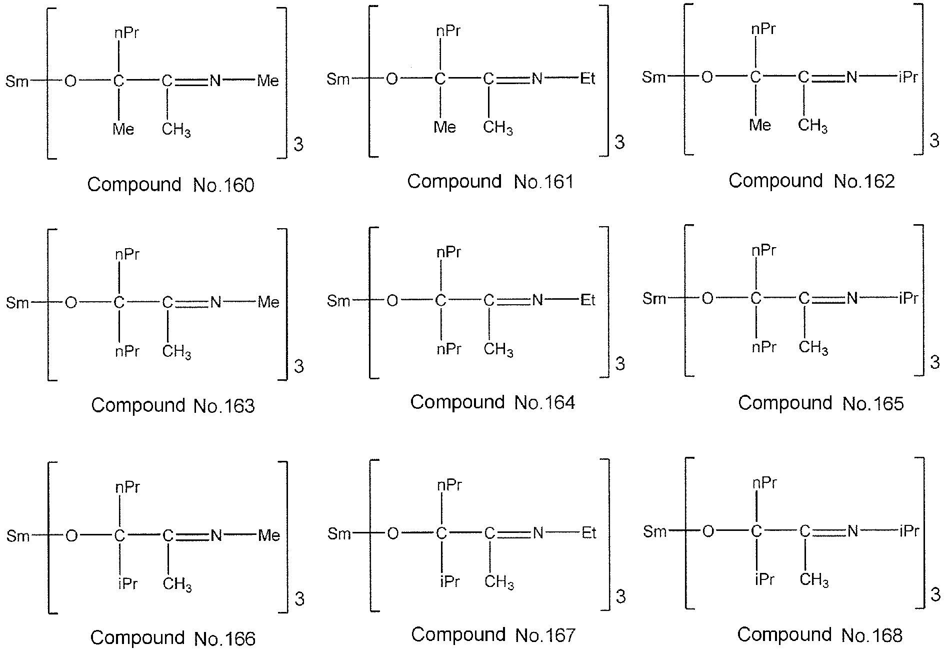

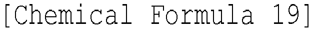

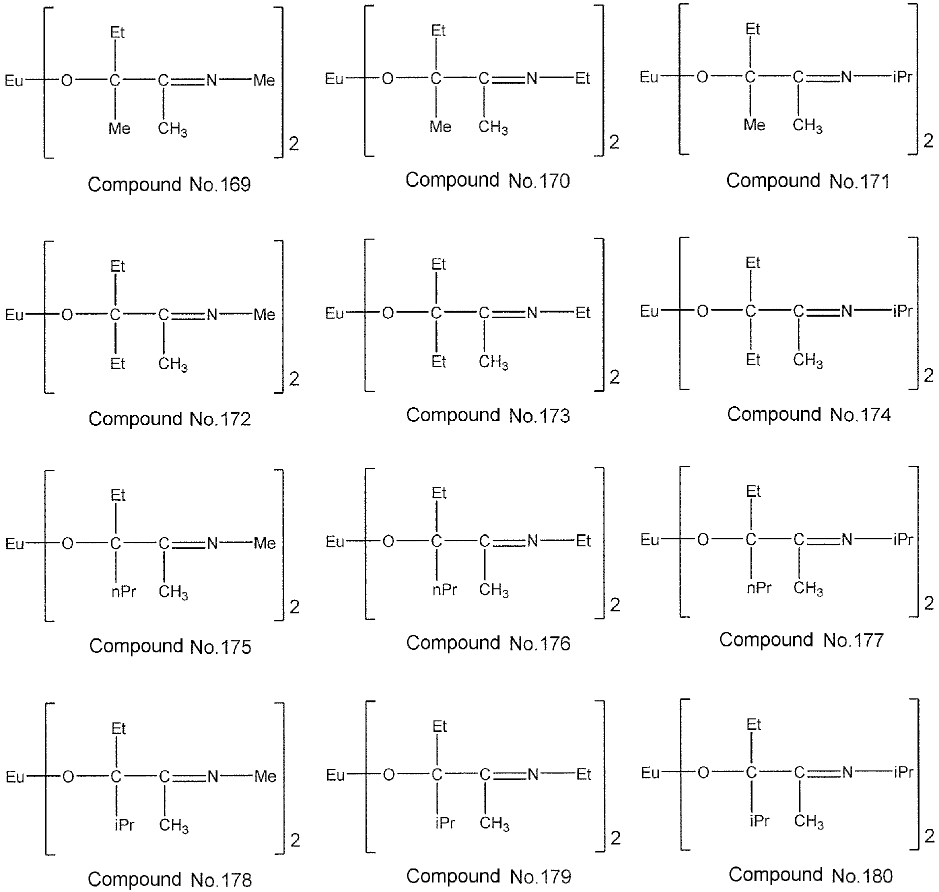

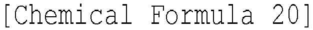

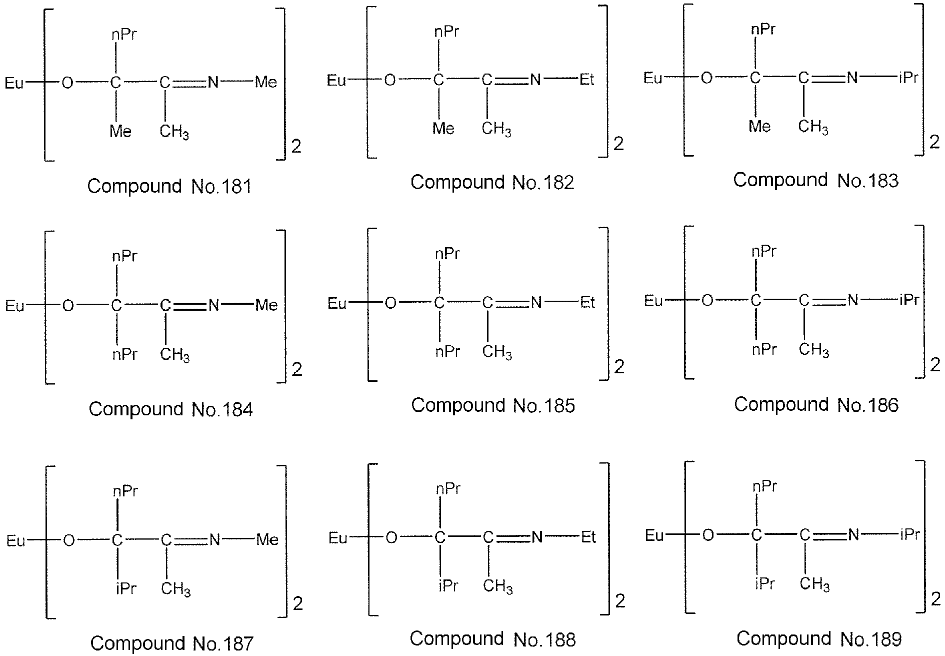

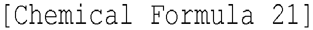

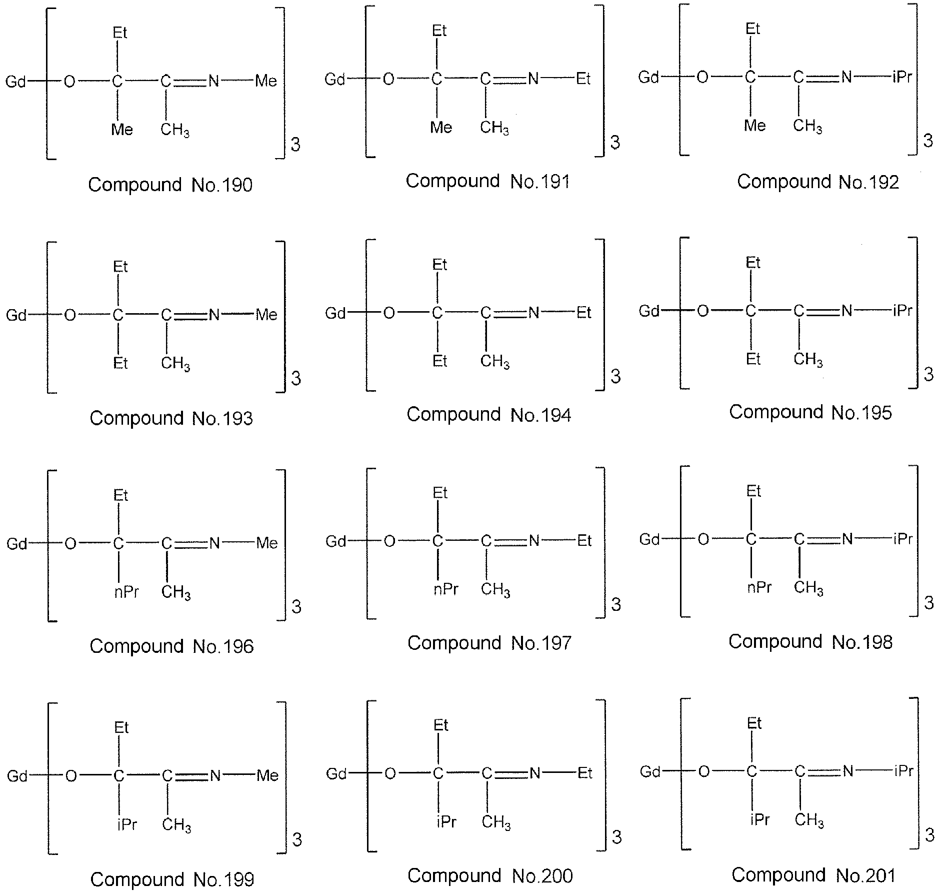

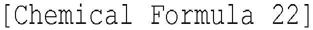

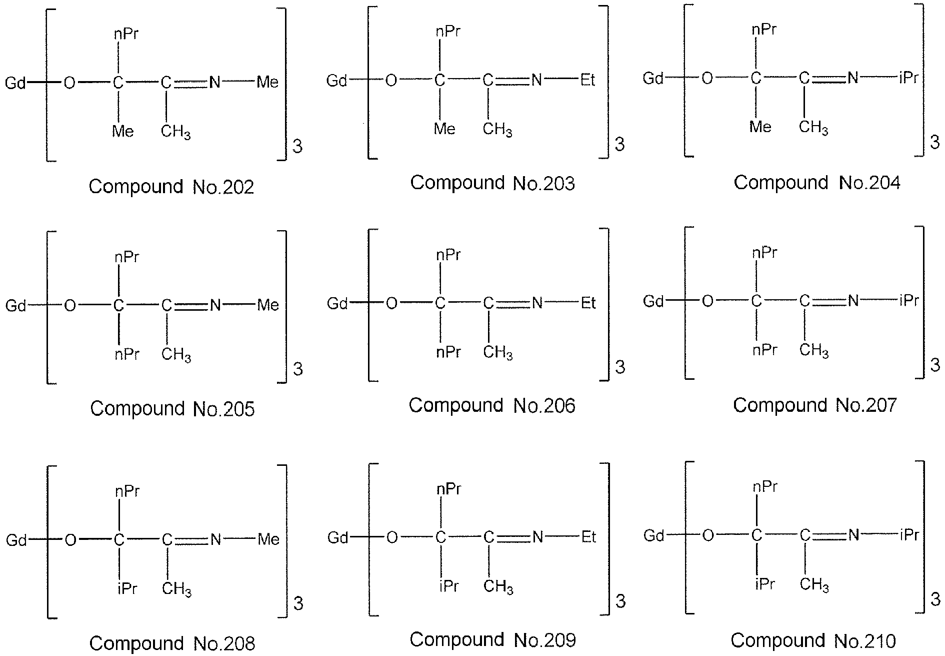

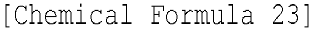

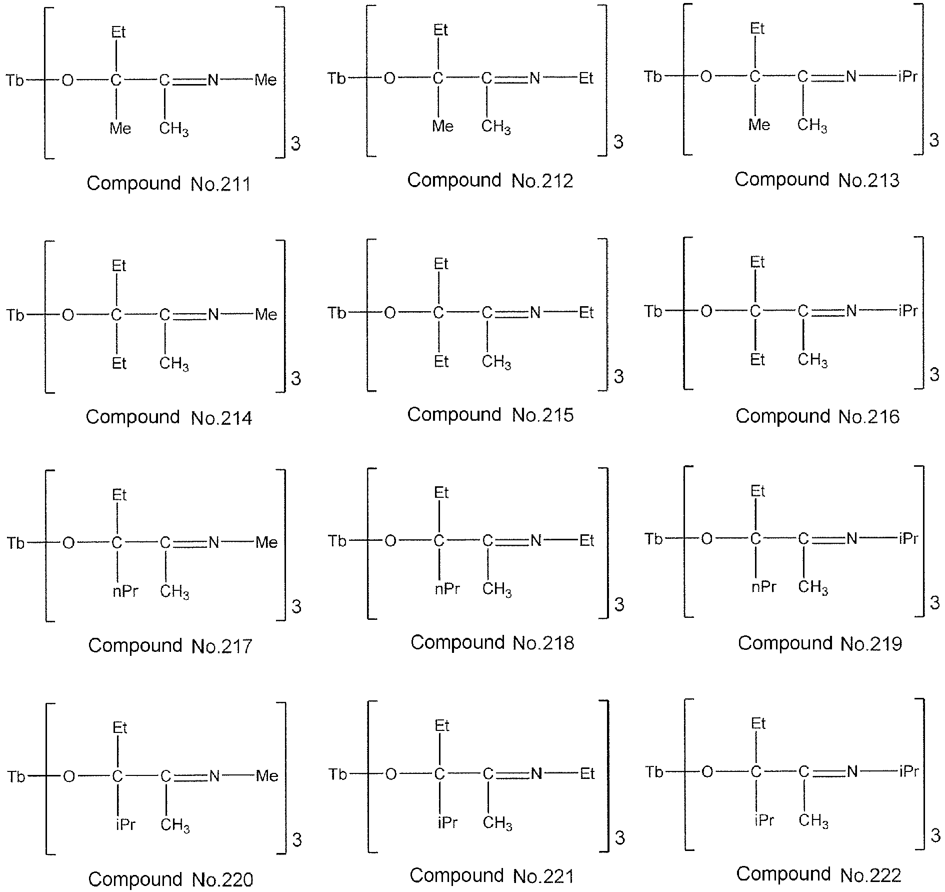

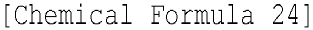

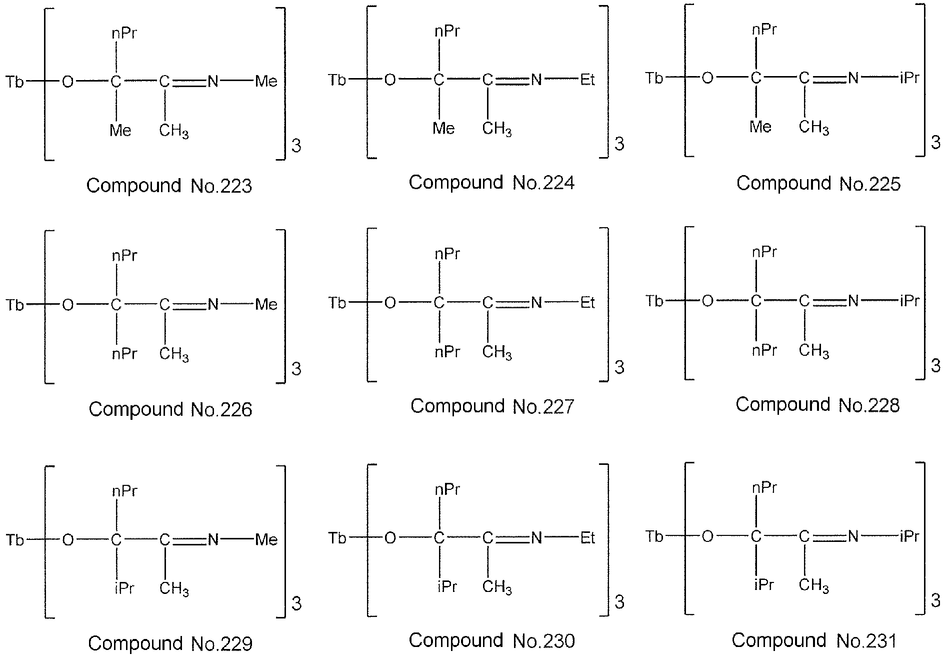

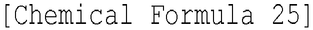

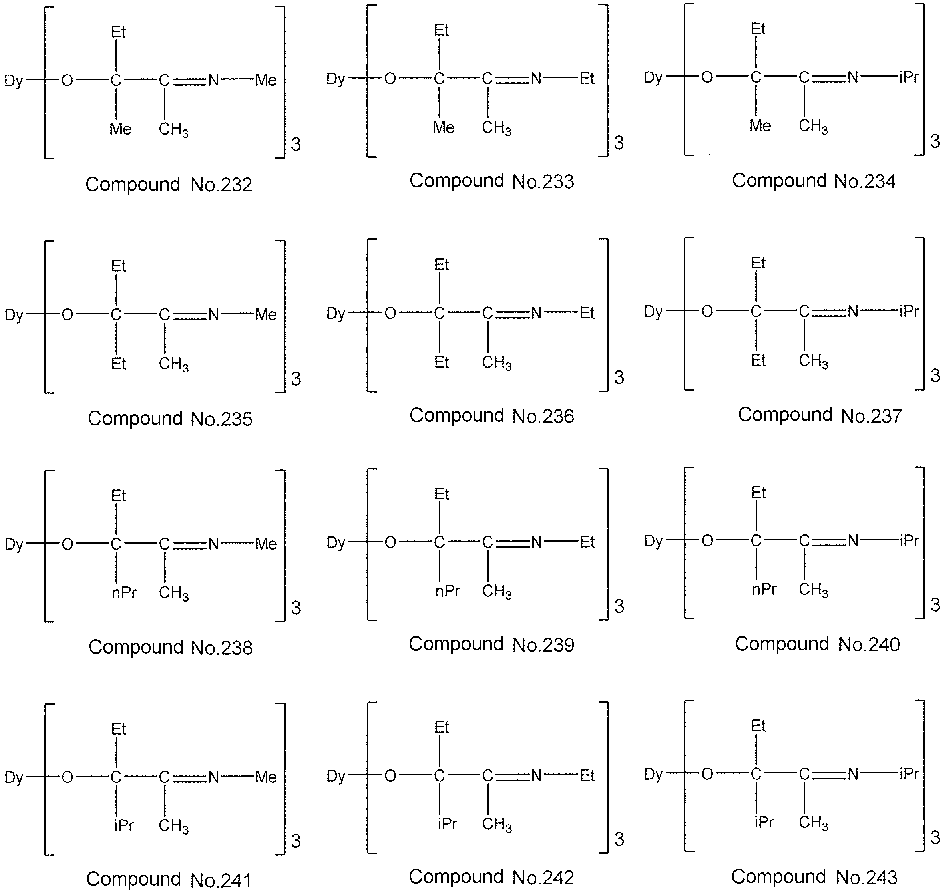

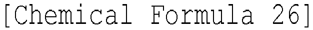

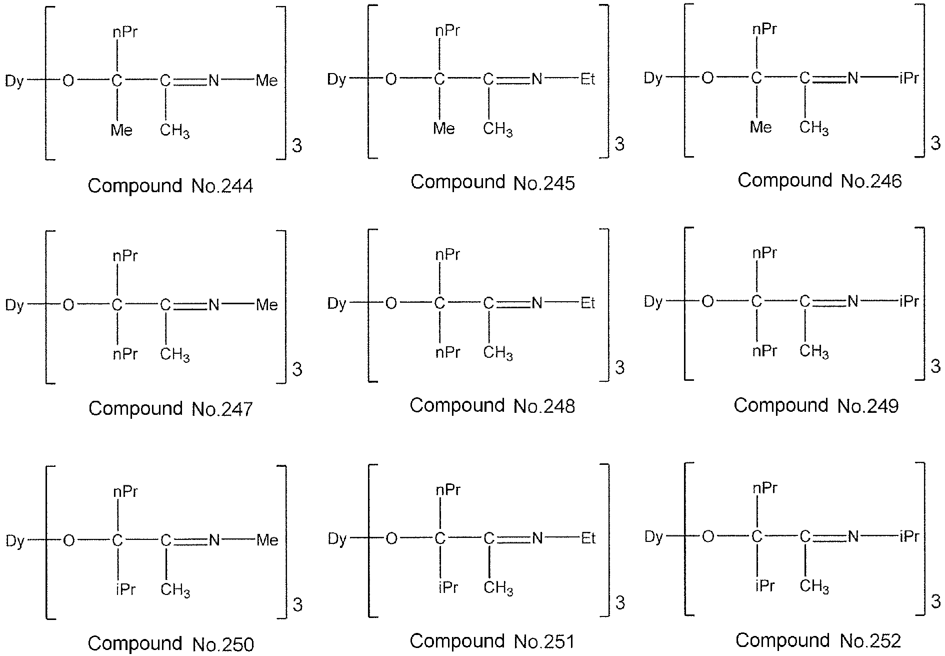

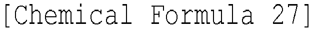

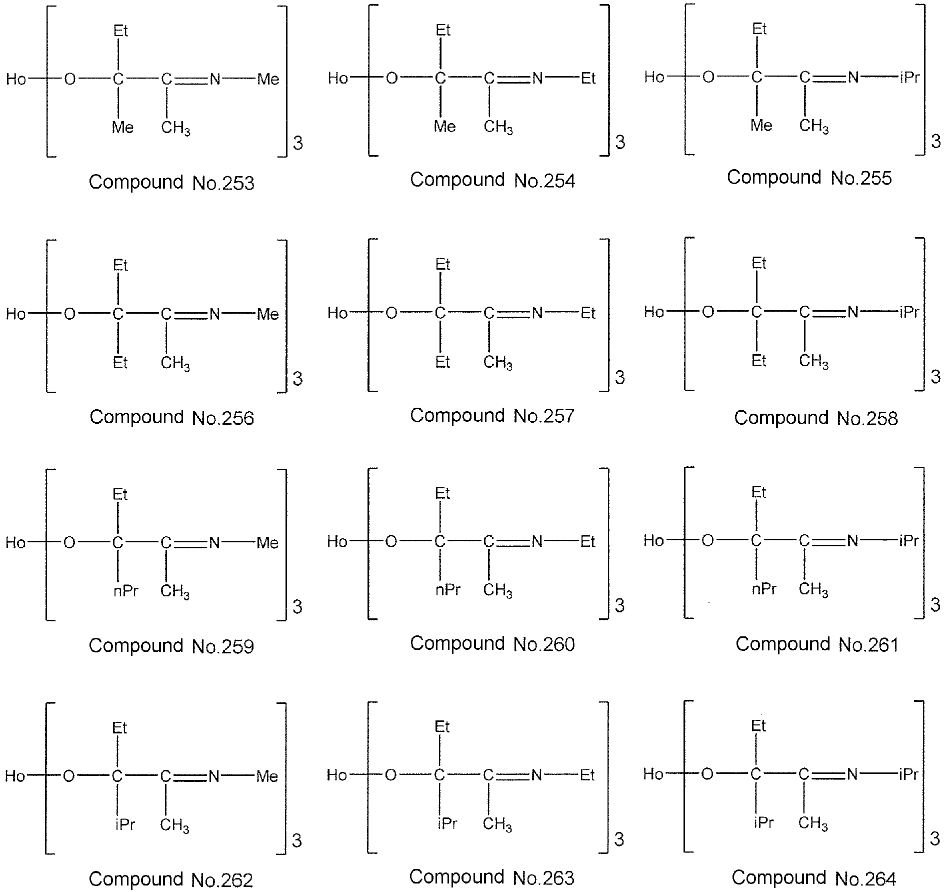

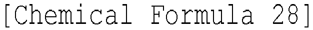

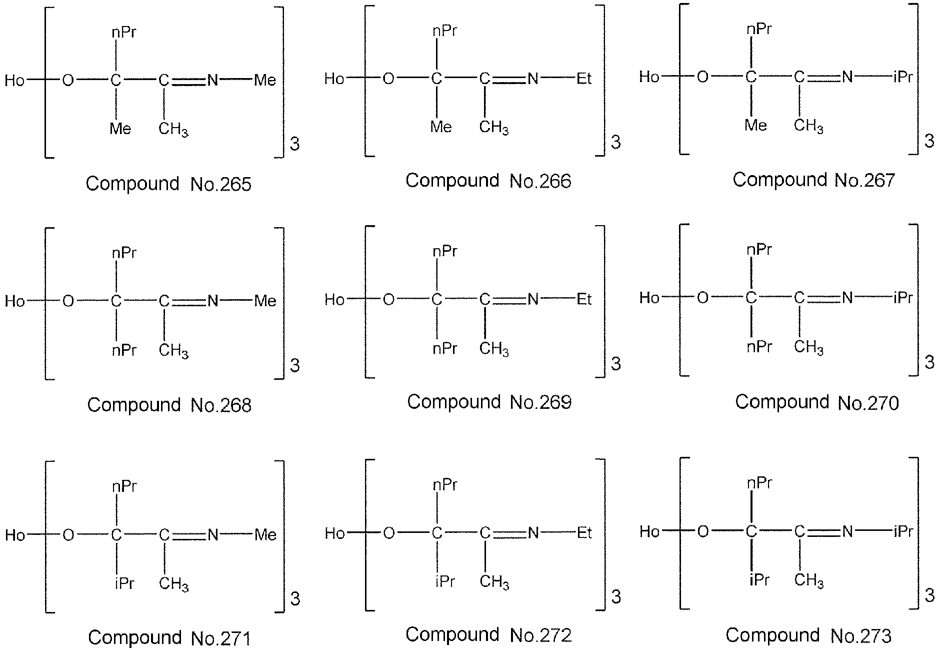

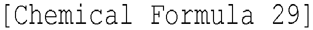

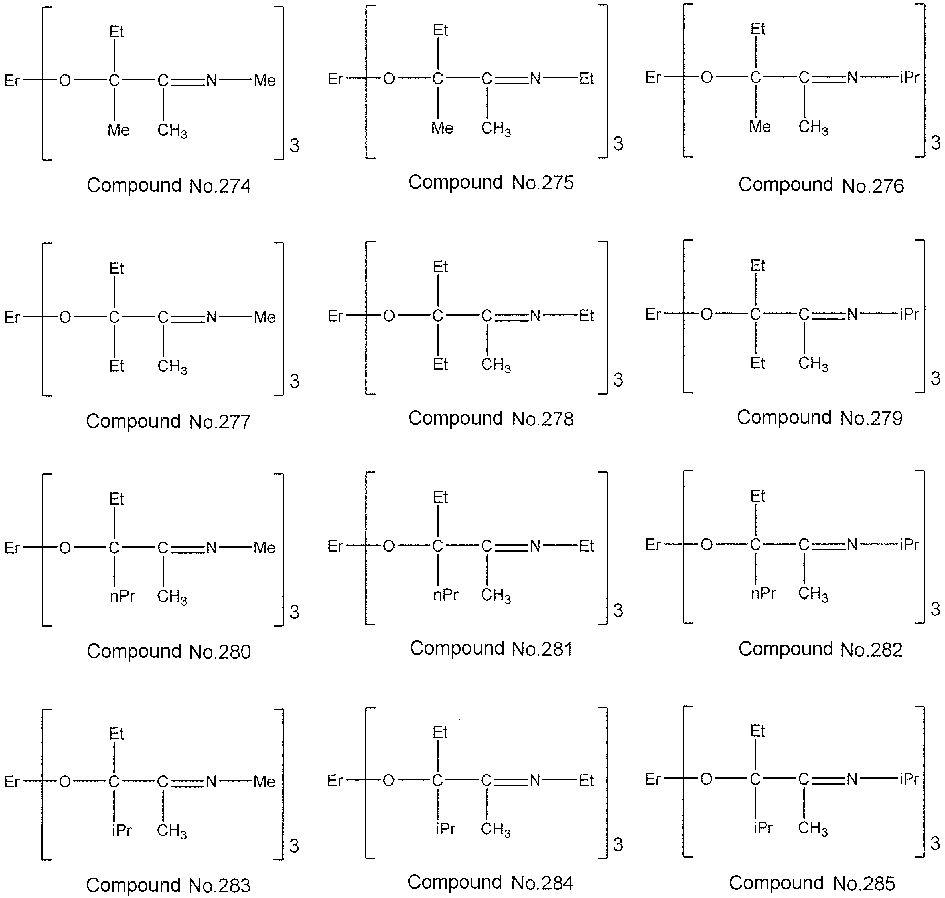

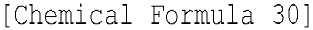

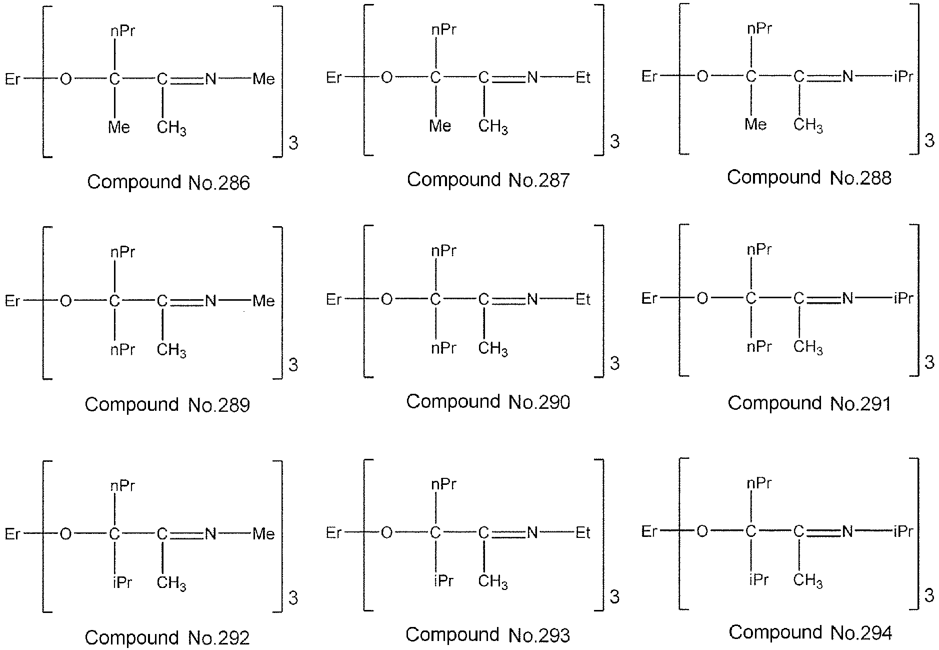

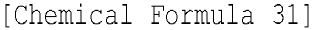

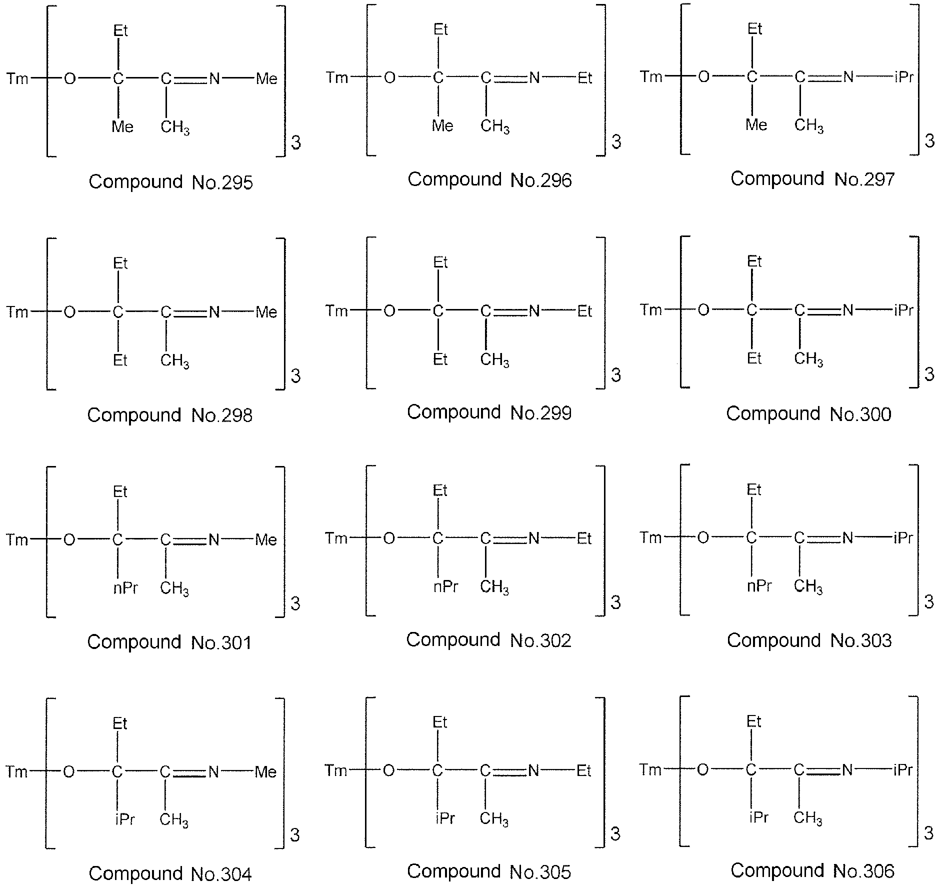

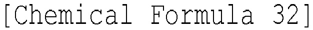

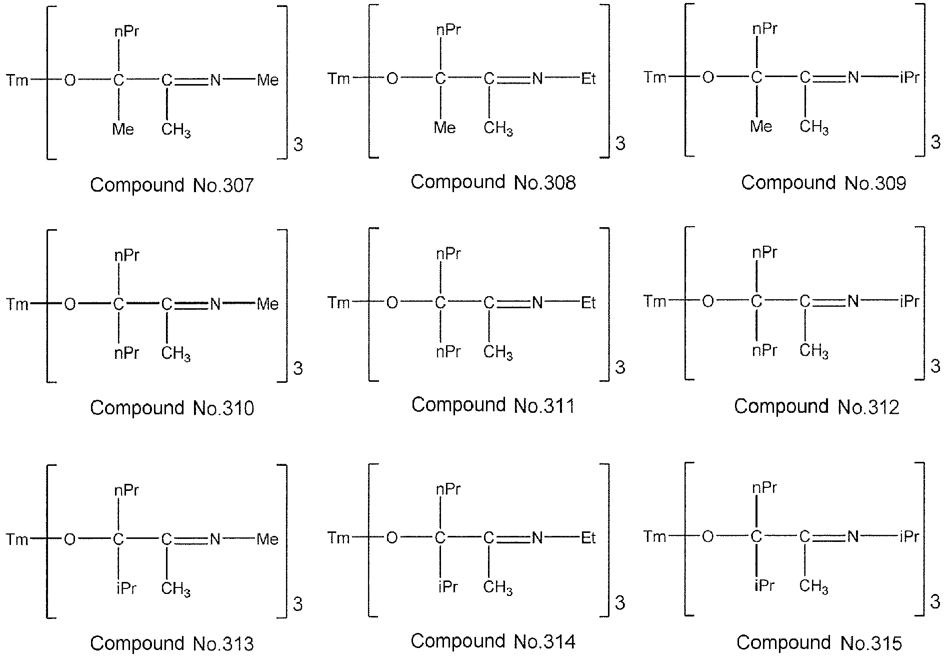

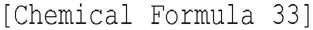

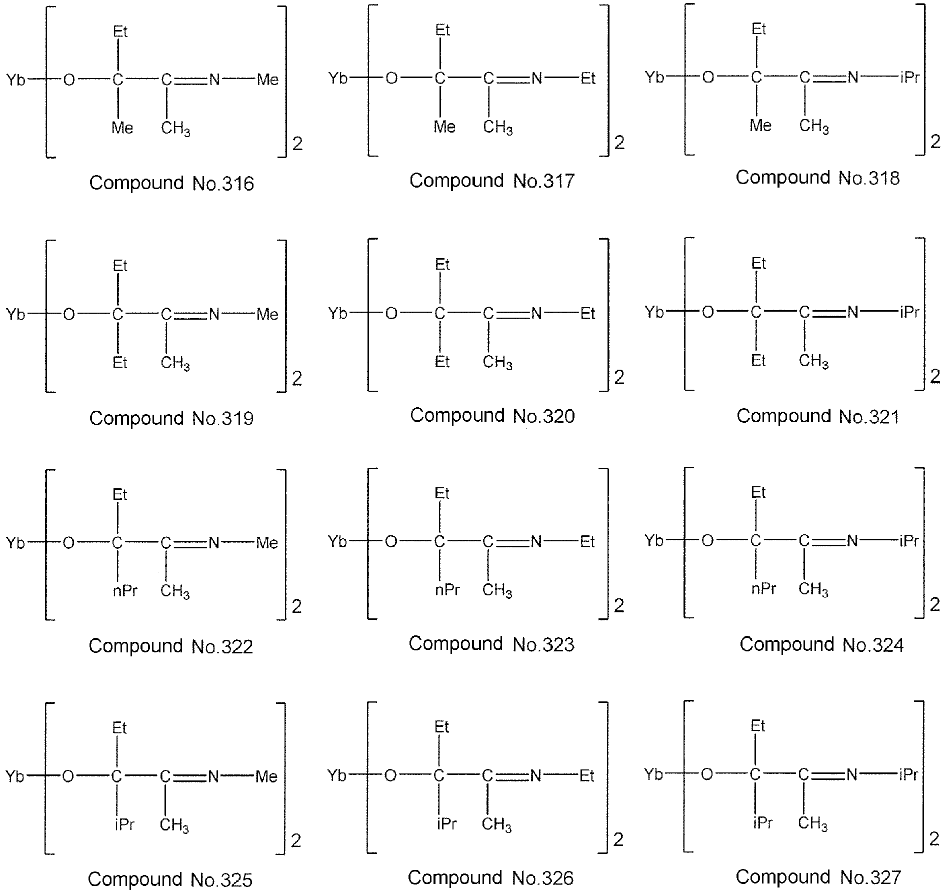

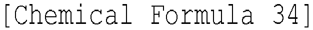

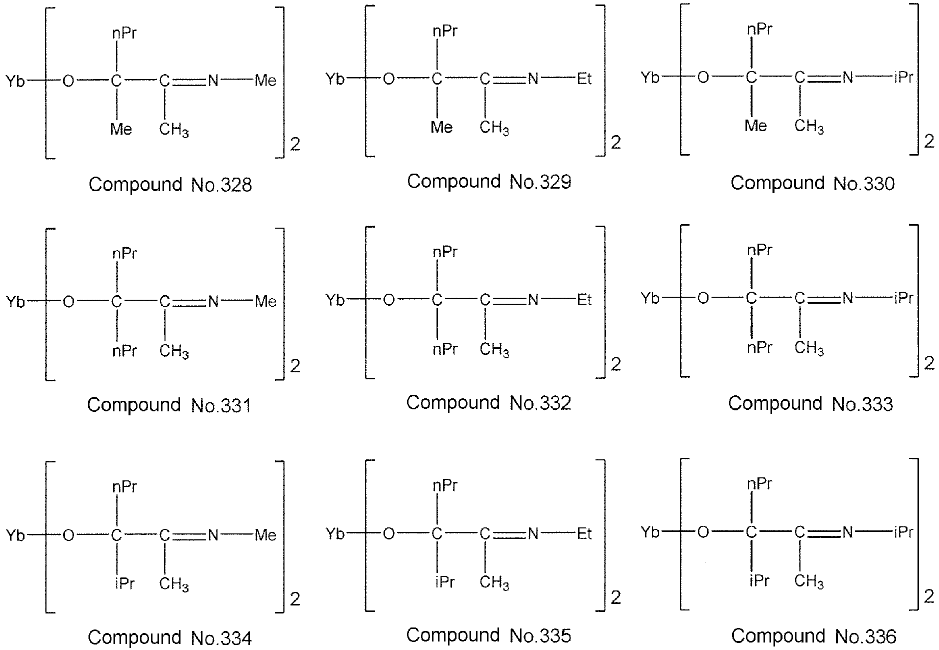

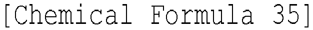

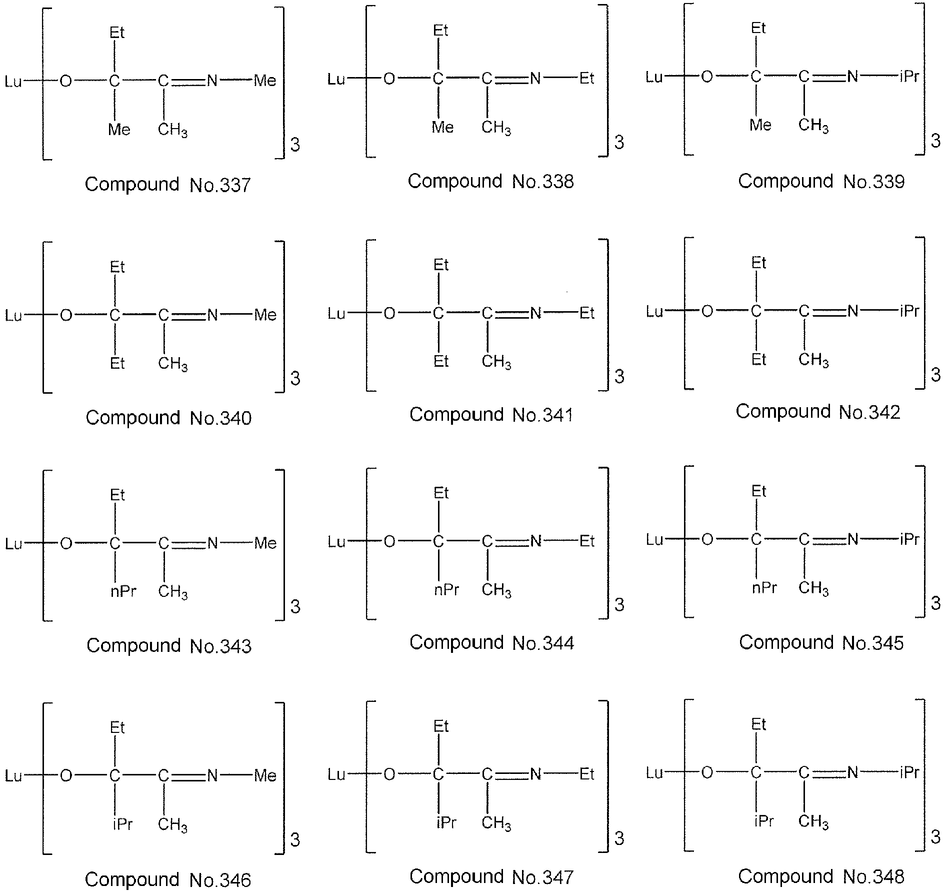

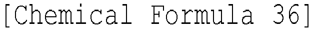

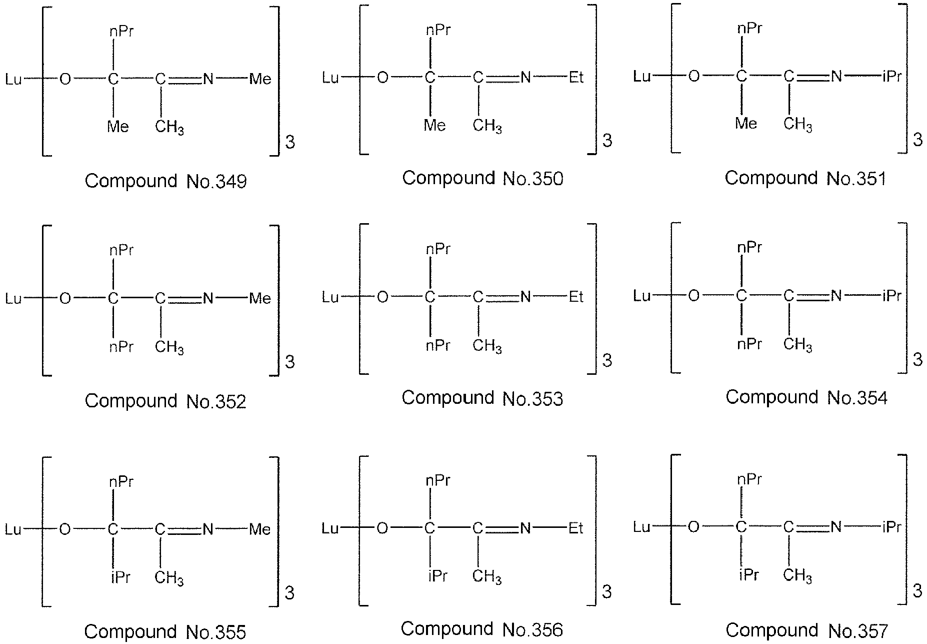

The metal alkoxide compound of the present invention is not particularly limited by the production method and can be produced by applying a known reaction. Regarding an alkoxide compound production method, a synthesis method of a generally known alkoxide compound using a corresponding alcohol, can be applied. For example, when a lutetium alkoxide compound is produced, a method in which, for example, an inorganic salt such as a lutetium halide, a nitrate, or its hydrate and a corresponding alcohol compound are reacted in the presence of a base such as sodium, sodium hydride, sodium amide, sodium hydroxide, sodium methylate, ammonia, and amine; a method in which inorganic salts such as lutetium halides, nitrates or their hydrates are reacted with an alkali metal alkoxide such as a sodium alkoxide, a lithium alkoxide, and a potassium alkoxide of a corresponding alcohol compound; a method in which a low-molecular-weight alcohol alkoxide compound such as lutetium methoxide, ethoxide, isopropoxide, and butoxide and a corresponding alcohol compound are subjected to an interchange reaction; and a method in which inorganic salts such as lutetium halides and nitrates are reacted with derivatives of the yield reactive intermediates to obtain reactive intermediates, and then the intermediates are reacted with a corresponding alcohol compound or the like may be used. Examples of reactive intermediates include bis(dialkylamino)lutetium, bis(bis(trimethylsilyl)amino)lutetium, and lutetium amide compounds.

Next, in a thin-film-forming raw material of the present invention, the metal alkoxide compound of the present invention described above is used as a precursor of a thin film, and its form differs depending on a production process in which the thin-film-forming raw material is applied. For example, when a thin film containing only at least one atom selected from among a scandium atom, an yttrium atom, a lanthanum atom, a cerium atom, a praseodymium atom, a neodymium atom, a promethium atom, a samarium atom, a europium atom, a gadolinium atom, a terbium atom, a dysprosium atom, a holmium atom, an erbium atom, a thulium atom, an ytterbium atom and a lutetium atom is produced, the thin-film-forming raw material of the present invention does not contain a metal compound other than the metal alkoxide compound and a semi-metal compound. On the other hand, when a thin film containing two or more metals and/or a semi-metal is produced, the thin-film-forming raw material of the present invention can contain, in addition to the above metal alkoxide compound, a compound containing a desired metal and/or a compound containing a semi-metal (hereinafter referred to as other precursors). As will be described below, the thin-film-forming raw material of the present invention may further contain an organic solvent and/or a nucleophilic reagent. The thin-film-forming raw material of the present invention is particularly useful as a raw material for chemical vapor deposition (hereinafter referred to as a "raw material for CVD") because, as described above, physical properties of the metal alkoxide compound as a precursor are suitable for the CVD method and the ALD method.

When the thin-film-forming raw material of the present invention is a raw material for CVD, its form can be appropriately selected by a method such as a transport supply method in the CVD method used or the like.

Regarding the transport supply method, a gas transport method in which a raw material for CVD is vaporized into vapor by being heated and/or depressurized in a container in which the raw material is stored (hereinafter simply referred to as a "raw material container"), and as necessary, together with a carrier gas such as argon, nitrogen, and helium used, the vapor is introduced into a film-forming chamber in which a substrate is provided (hereinafter referred to as a "deposition reaction unit") and a liquid transport method in which a raw material for CVD in a liquid or a solution state is transported to a vaporization chamber and is vaporized into vapor by being heated and/or depressurized in the vaporization chamber, and the vapor is introduced into a film-forming chamber may be exemplified. In the case of the gas transport method, the metal alkoxide compound represented by general formula (I) can be directly used as a raw material for CVD. In the case of the liquid transport method, the metal alkoxide compound itself represented by general formula (I) or a solution in which the compound is dissolved in an organic solvent can be used as a raw material for CVD. Such a CVD raw material may further contain other precursors, a nucleophilic reagent, and the like.

In addition, in the multi-component CVD method, a method in which components in a raw material for CVD are independently vaporized and supplied (hereinafter referred to as a "single source method") and a method in which a mixed raw material in which multi-component raw materials are mixed in a desired composition in advance is vaporized and supplied (hereinafter referred to as a "cocktail source method") are used. In the case of the cocktail source method, a mixture containing the metal alkoxide compound of the present invention and other precursors or a mixed solution in which the mixture is dissolved in an organic solvent can be used as a raw material for CVD. The mixture or mixed solution may further contain a nucleophilic reagent and the like. Here, when only the metal alkoxide compound of the present invention is used as a precursor and an R form and an S form are used together, a raw material for CVD including an R form and a raw material for CVD including an S form may be separately vaporized or a raw material for CVD including a mixture of an R form and an S form may be vaporized.

Regarding the organic solvent, a generally known organic solvent can be used without any particular limitation. Examples of organic solvents include acetate esters such as ethyl acetate, butyl acetate, and methoxyethyl acetate; ethers such as tetrahydrofuran, tetrahydropyran, ethylene glycol dimethyl ether, diethylene glycol dimethyl ether, triethylene glycol dimethyl ether, dibutyl ether, and dioxane; ketones such as methyl butyl ketone, methyl isobutyl ketone, ethyl butyl ketone, dipropyl ketone, diisobutyl ketone, methyl amyl ketone, cyclohexanone, and methyl cyclohexanone; hydrocarbons such as hexane, cyclohexane, methylcyclohexane, dimethylcyclohexane, ethylcyclohexane, heptane, octane, toluene, and xylene; hydrocarbons having a cyano group such as 1-cyanopropane, 1-cyanobutane, 1-cyanohexane, cyanocyclohexane, cyanobenzene, 1,3-dicyanopropane, 1,4-dicyanobutane, 1,6-dicyanohexane, 1,4-dicyanocyclohexane, and 1,4-dicyanobenzene; pyridine; and lutidine. These may be used alone or as a solvent in which two or more thereof are mixed depending on the solubility of a solute, the relationship between an operation temperature, a boiling point, and a flash point, and the like. When such an organic solvent is used, preferably, a total amount of the precursors in a raw material for CVD, which is a solution in which the precursors are dissolved in the organic solvent, is 0.01 to 2.0 mol/liter, and particularly 0.05 to 1.0 mol/liter. When the thin-film-forming raw material of the present invention does not contain a metal compound other than the metal alkoxide compound of the present invention and a semi-metal compound, a total amount of the precursors is an amount of the metal alkoxide compound of the present invention, and when the thin-film-forming raw material of the present invention contains a compound containing another metal in addition to the metal alkoxide compound and/or a compound containing a semi-metal, a total amount of the precursors is a total amount of the metal alkoxide compound of the present invention and other precursors.

In addition, in the case of the multi-component CVD method, regarding other precursors to be used together with the metal alkoxide compound of the present invention, a generally known precursor used in a raw material for CVD can be used without any particular limitation.

Regarding the other precursors, compounds including one or two or more selected from the group consisting of compounds used as organic ligands such as an alcohol compound, a glycol compound, a β-diketone compound, a cyclopentadiene compound, and an organic amine compound, and silicon and a metal may be exemplified. In addition, examples of the type of a metal of the precursor include lithium, sodium, potassium, magnesium, calcium, strontium, barium, titanium, zirconium, hafnium, vanadium, niobium, tantalum, chromium, molybdenum, tungsten, manganese, iron, ruthenium, cobalt, rhodium, iridium, nickel, palladium, platinum, copper, silver, gold, zinc, aluminum, gallium, indium, germanium, tin, lead, antimony, and bismuth.

Examples of an alcohol compound used as an organic ligand of the other precursor include alkyl alcohols such as methanol, ethanol, propanol, isopropyl alcohol, butanol, secondary butyl alcohol, isobutyl alcohol, tertiary butyl alcohol, pentyl alcohol, isopentyl alcohol, and tertiary pentyl alcohol; ether alcohols such as 2-methoxyethanol, 2-ethoxyethanol, 2-butoxyethanol, 2-(2-methoxyethoxy)ethanol, 2-methoxy-1-methylethanol, 2-methoxy-1,1-dimethylethanol, 2-ethoxy-1,1-dimethylethanol, 2-isopropoxy-1,1-dimethylethanol, 2-butoxy-1,1-dimethylethanol, 2-(2-methoxyethoxy)-1,1-dimethylethanol, 2-propoxy-1,1-diethylethanol, 2-s-butoxy-1,1-diethylethanol, and 3-methoxy-1,1-dimethylpropanol; and dialkylamino alcohols such as dimethylaminoethanol, ethylmethylaminoethanol, diethylaminoethanol, dimethylamino-2-pentanol, ethylmethylamino-2-pentanol, dimethylamino-2-methyl-2-pentanol, ethyl methylamino-2-methyl-2-pentanol, and diethylamino-2-methyl-2-pentanol.

Examples of a glycol compound used as an organic ligand of the other precursor include 1,2-ethanediol, 1,2-propanediol, 1,3-propanediol, 2,4-hexanediol, 2,2-dimethyl-1,3-propanediol, 2,2-diethyl-1,3-propanediol, 1,3-butanediol, 2,4-butanediol, 2,2-diethyl-1,3-butanediol, 2-ethyl-2-butyl-1,3-propanediol, 2,4-pentanediol, 2-methyl-1,3-propanediol, 2-methyl-2,4-pentanediol, 2,4-hexanediol, and 2,4-dimethyl-2,4-pentanediol.

In addition, examples of β-diketone compounds include alkyl-substituted β-diketones such as acetylacetone, hexane-2,4-dione, 5-methylhexane-2,4-dione, heptane-2,4-dione, 2-methylheptane-3,5-dione, 5-methylheptane-2,4-dione, 6-methylheptane-2,4-dione, 2,2-dimethylheptane-3,5-dione, 2,6-dimethylheptane-3,5-dione, 2,2,6-trimethylheptane-3,5-dione, 2,2,6,6-tetramethylheptane-3,5-dione, octane-2,4-dione, 2,2,6-trimethyloctane-3,5-dione, 2,6-dimethyloctane-3,5-dione, 2,9-dimethylnonane-4,6-dione, 2-methyl-6-ethyldecane-3,5-dione, and 2,2-dimethyl-6-ethyldecane-3,5-dione; fluorine-substituted alkyl β-diketones such as 1,1,1-trifluoropentane-2,4-dione, 1,1,1-trifluoro-5,5-dimethylhexane-2,4-dione, 1,1,1,5,5,5-hexafluoropentane-2,4-dione, and 1,3-diperfluorohexylpropane-1,3-dione; and ether-substituted β-diketones such as 1,1,5,5-tetramethyl-1-methoxyhexane-2,4-dione, 2,2,6,6-tetramethyl-1-methoxyheptane-3,5-dione, and 2,2,6,6-tetramethyl-1-(2-methoxyethoxy)heptane-3,5-dione.

In addition, examples of cyclopentadiene compounds include cyclopentadiene, methylcyclopentadiene, ethylcyclopentadiene, propylcyclopentadiene, isopropylcyclopentadiene, butylcyclopentadiene, secondary butylcyclopentadiene, isobutyl cyclopentadiene, tertiary butylcyclopentadiene, dimethyl cyclopentadiene, and tetramethyl cyclopentadiene, and examples of organic amine compounds used as the organic ligand include methylamine, ethylamine, propylamine, isopropylamine, butylamine, secondary butylamine, tertiary butylamine, isobutylamine, dimethylamine, diethylamine, dipropylamine, diisopropylamine, ethylmethylamine, propylmethylamine, and isopropylmethylamine.

The other precursors are known in the related art and methods of producing the same are known. Regarding a production method example, for example, when an alcohol compound is used as an organic ligand, an inorganic salt of a metal or its hydrate described above is reacted with an alkali metal alkoxide of the alcohol compound, and thereby a precursor can be produced. Here, examples of an inorganic salt of a metal or its hydrate include metal halides and nitrates, and examples of alkali metal alkoxides include sodium alkoxide, lithium alkoxide, and potassium alkoxide.

In the case of a single source method, the other precursor is preferably a compound having a similar thermal and/or oxidative decomposition behavior to the metal alkoxide compound of the present invention, and in the case of a cocktail source method, a compound which has a similar thermal and/or oxidative decomposition behavior and also does not cause deterioration due to a chemical reaction or the like during mixing is preferable.

In addition, the thin-film-forming raw material of the present invention may contain, as necessary, a nucleophilic reagent for imparting stability to the metal alkoxide compound of the present invention and other precursors. Examples of nucleophilic reagents include ethylene glycol ethers such as glyme, diglyme, triglyme, and tetraglyme; crown ethers such as 18-crown-6, dicyclohexyl-18-crown-6, 24-crown-8, dicyclohexyl-24-crown-8, and dibenzo-24-crown-8; polyamines such as ethylenediamine, N,N'-tetramethylethylenediamine, diethylenetriamine, triethylenetetramine, tetraethylenepentamine, pentaethylenehexamine, 1,1,4,7,7-pentamethyldiethylenetriamine, 1,1,4,7,10,10-hexamethyltriethylenetetramine, and triethoxytriethyleneamine; cyclic polyamines such as cyclam and cyclen; heterocyclic compounds such as pyridine, pyrrolidine, piperidine, morpholine, N-methyl pyrrolidine, N-methyl piperidine, N-methyl morpholine, tetrahydrofuran, tetrahydropyran, 1,4-dioxane, oxazole, thiazole, and oxathiolane; β-ketoesters such as methyl acetoacetate, ethyl acetoacetate, and 2-methoxyethyl acetoacetate; and β-diketones such as acetylacetone, 2,4-hexanedione, 2,4-heptanedione, 3,5-heptanedione, and dipivaloylmethane. An amount of such a nucleophilic reagent used is preferably in a range of 0.1 to 10 mol and more preferably in a range of 1 to 4 mol with respect to 1 mol of a total amount of the precursors.

In the thin-film-forming raw material of the present invention, an impurity metal element content, an impurity halogen content such as impurity chlorine, and an impurity organic component other than components constituting the same are minimized. The impurity metal element content is preferably 100 ppb or less and more preferably 10 ppb or less for each element, and a total amount thereof is preferably 1 ppm or less and more preferably 100 ppb or less. In particular, when used as a gate insulating film, a gate film, or a barrier layer of LSI, it is necessary to reduce the content of alkali metal elements, alkaline earth metal elements, and analogous elements which affect electrical characteristics of the obtained thin film. The impurity halogen content is preferably 100 ppm or less, more preferably 10 ppm or less, and most preferably 1 ppm or less. A total amount of the impurity organic component is preferably 500 ppm or less, more preferably 50 ppm or less, and most preferably 10 ppm or less. In addition, since water causes generation of particles in the raw material for chemical vapor deposition and generation of particles during thin film formation, in order to reduce water in each of the metal compound, the organic solvent, and the nucleophilic reagent, it is preferable to remove water as much as possible during use. The content of water in each of the metal compound, the organic solvent and the nucleophilic reagent is preferably 10 ppm or less and more preferably 1 ppm or less.

In addition, in the thin-film-forming raw material of the present invention, in order to reduce or prevent particle contaminants in the formed thin film, it is preferable that as few particles as possible be contained. Specifically, in measurement of particles by a particle detector in a light scattering liquid in a liquid phase, the number of particles larger than 0.3 µm is preferably 100 or less in 1 ml of a liquid phase, the number of particles larger than 0.2 µm is more preferably 1,000 or less in 1 ml of a liquid phase, and the number of particles larger than 0.2 µm is most preferably 100 or less in 1 ml of a liquid phase.

The method of producing a thin film of the present invention in which a thin film is produced using the thin-film-forming raw material of the present invention is a CVD method in which vapor obtained by vaporizing the thin-film-forming raw material of the present invention and a reactive gas used as necessary are introduced into a film-forming chamber in which a substrate is provided (processing atmosphere), and next, a precursor is decomposed and/or chemically reacted on the substrate and a metal-containing thin film grows and is deposited on the surface of the substrate. Regarding raw material transport supply methods, deposition methods, production conditions, production devices, and the like, generally known conditions and methods can be used without any particular limitation.

Examples of reactive gases used as necessary include oxygen, ozone, nitrogen dioxide, nitrogen monooxide, water vapor, hydrogen peroxide, formic acid, acetic acid, and acetic anhydride which have oxidizability and hydrogen which has reducibility, and examples of those for producing nitrides include organic amine compounds such as monoalkylamine, dialkylamine, trialkylamine, and alkylenediamine; hydrazine; and ammonia, and one, two or more thereof can be used. Among these, since the thin-film-forming raw material of the present invention has favorable reactivity with ozone, when one reactive gas is used, ozone is preferably used, and when a gas in which two or more thereof are mixed is used as a reactive gas, it is preferable to include at least ozone.

In addition, examples of transport supply methods include the gas transport method, liquid transport method, single source method, and cocktail source method described above.

In addition, examples of deposition methods include thermal CVD in which a thin film is deposited by reacting a raw material gas or a raw material gas and a reactive gas only by heating, plasma CVD using heat and plasma, photo CVD using heat and light, light plasma CVD using heat, light and plasma, and ALD in which a CVD deposition reaction is divided into elementary processes and stepwise deposition is performed at the molecular level.

Examples of materials of the substrate include silicon; ceramics such as silicon nitride, titanium nitride, tantalum nitride, titanium oxide, titanium nitride, ruthenium oxide, zirconium oxide, hafnium oxide, and lanthanum oxide; glass; and metals such as metal ruthenium. Examples of shapes of the substrate include a plate shape, a spherical shape, a fibrous shape, and a scaly shape. The surface of the substrate may be flat or may have a three-dimensional structure such as a trench structure.

In addition, the production conditions include a reaction temperature (substrate temperature), a reaction pressure, a deposition rate, and the like. The reaction temperature is preferably 100°C or higher which is a temperature at which the metal alkoxide compound of the present invention sufficiently reacts, and more preferably 150°C to 400°C, and particularly preferably 200°C to 350°C. In addition, the reaction pressure is preferably 10 Pa to atmospheric pressure in the case of the thermal CVD or photo CVD, and is preferably 10 Pa to 2, 000 Pa when plasma is used.

In addition, the deposition rate can be controlled according to a raw material supply condition (vaporization temperature, vaporization pressure), a reaction temperature, and a reaction pressure. The deposition rate is preferably 0.01 nm/min to 100 nm/min and more preferably 1 nm/min to 50 nm/min because characteristics of the obtained thin film may deteriorate if the deposition rate is large and problems may occur in productivity if the deposition rate is small. In addition, in the ALD method, the number of cycles is controlled to obtain a desired film thickness.

The production conditions further include a temperature and pressure at which the thin-film-forming raw material is vaporized into vapor. A process of vaporizing the thin-film-forming raw material into vapor may be performed in the raw material container or in the vaporization chamber. In any case, the thin-film-forming raw material of the present invention is preferably evaporated at 0°C to 150°C. In addition, when the thin-film-forming raw material is vaporized into vapor in the raw material container or the vaporization chamber, the pressure in the raw material container and the pressure in the vaporization chamber both are preferably 1 Pa to 10,000 Pa.

The method of producing a thin film of the present invention adopts the ALD method, and may include, in addition to a raw material introducing process in which a thin-film-forming raw material is vaporized into vapor, and the vapor is introduced into a film-forming chamber by the transport supply method, a precursor thin film forming process in which a precursor thin film is formed on the surface of the substrate using the compound in the vapor, an exhaust process in which an unreacted compound gas is exhausted, and a metal-containing thin film forming process in which the precursor thin film is chemically reacted with a reactive gas to form a metal-containing thin film on the surface of the substrate.

Hereinafter, respective processes of the ALD method will be described in detail using a case in which a metal oxide thin film is formed as an example. First, the above raw material introducing process is performed. A preferable temperature and pressure when a thin-film-forming raw material is vaporized are the same as those described in the method of producing a thin film according to the CVD method. Next, the vapor introduced into the film-forming chamber is in contact with the surface of the substrate, and thereby a precursor thin film is formed on the surface of the substrate (precursor thin film forming process) . In this case, the substrate may be heated or the film-forming chamber may be heated to apply heat. The precursor thin film formed in this process is a thin film formed from the metal alkoxide compound of the present invention or a thin film formed by decomposition and/or reaction of a part of the metal alkoxide compound of the present invention, and has a composition different from that of a desired metal oxide thin film. A substrate temperature when this process is performed is preferably room temperature to 500°C and more preferably 150°C to 350°C. The pressure of the system (in the film-forming chamber) in which this process is performed is preferably 1 Pa to 10,000 Pa and more preferably 10 Pa to 1,000 Pa.

Next, an unreacted compound gas and a byproduct gas are exhausted from the film-forming chamber (exhaust process). An unreacted compound gas and a byproduct gas should ideally be completely exhausted from the film-forming chamber, but they are not necessarily completely exhausted. Examples of an exhaust method include a method in which the inside of the system is purged with an inert gas such as nitrogen, helium, and argon, a method of exhausting by reducing the pressure in the system, and a combination method thereof. When the pressure is reduced, the degree of pressure reduction is preferably 0.01 Pa to 300 Pa and more preferably 0.01 Pa to 100 Pa.

Next, an oxidizing gas as a reactive gas is introduced into the film-forming chamber, and a metal oxide thin film is formed from the precursor thin film obtained in the previous precursor thin film forming process according to the action of the oxidizing gas or the action of the oxidizing gas and heat (metal-oxide-containing thin film forming process). A temperature when heat is applied in this process is preferably room temperature to 500°C and more preferably 150°C to 350°C. The pressure in the system (in the film-forming chamber) in which this process is performed is preferably 1 Pa to 10,000 Pa and more preferably 10 Pa to 1,000 Pa. Since the metal alkoxide compound of the present invention has favorable reactivity with an oxidizing gas, it is possible to obtain a metal oxide thin film having a small residual carbon content and high quality.

In the method of producing a thin film of the present invention, when the above ALD method is adopted, thin film deposition according to a series of operations including the above raw material introducing process, precursor thin film forming process, exhaust process and metal-oxide-containing thin film forming process is set as one cycle, and this cycle may be repeated a plurality of times until a thin film with a required film thickness is obtained. In this case, after one cycle is performed, in the same manner as in the exhaust process, an unreacted compound gas and a reactive gas (an oxidizing gas when a metal oxide thin film is formed), and additionally a byproduct gas are exhausted from the deposition reaction unit and the next one cycle is then preferably performed.

In addition, in formation of a metal oxide thin film according to the ALD method, an energy such as plasma, light, and a voltage may be applied or a catalyst may be used. A time for which the energy is applied and a time for which a catalyst is used are not particularly limited, and the time may be, for example, during introduction of a compound gas in the raw material introducing process, during heating in the precursor thin film forming process or in the metal-oxide-containing thin film forming process, during exhaust of the system in the exhaust process, or during introduction of an oxidizing gas in the metal-oxide-containing thin film forming process, or may be between the processes.

In addition, in the method of producing a thin film of the present invention, after thin film deposition, in order to obtain more favorable electrical characteristics, an annealing treatment may be performed under an inert atmosphere, an oxidizing atmosphere, or reducing atmosphere, and a reflow process may be provided when step coverage is necessary. The temperature in this case is 200°C to 1,000°C and preferably 250°C to 500°C.

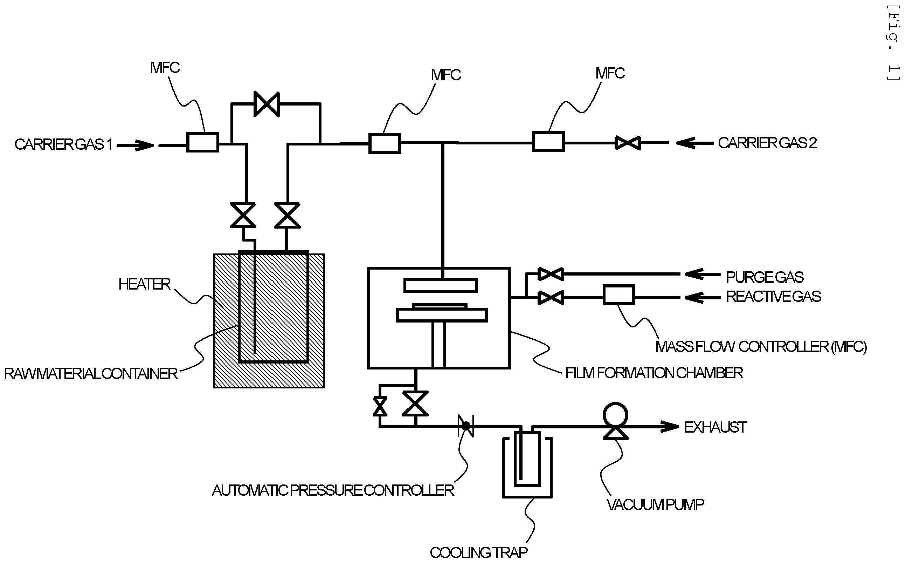

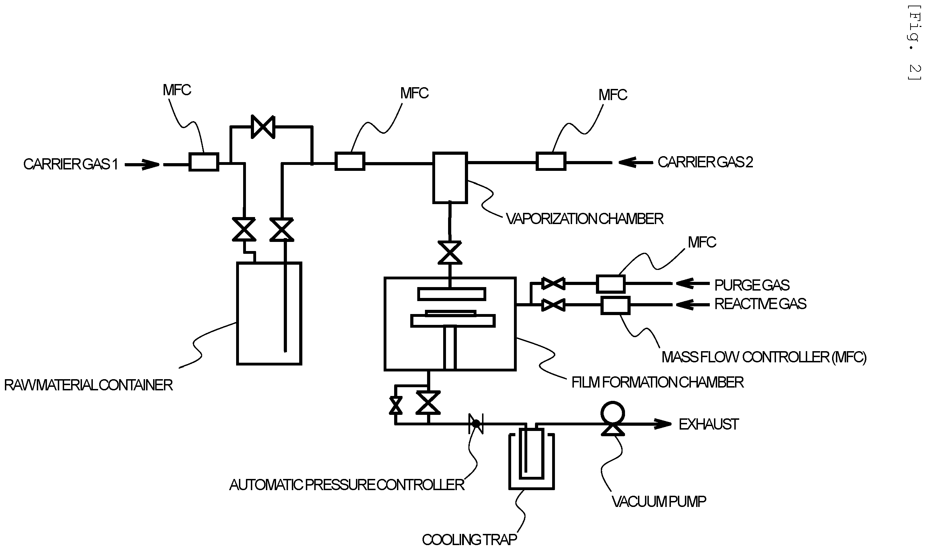

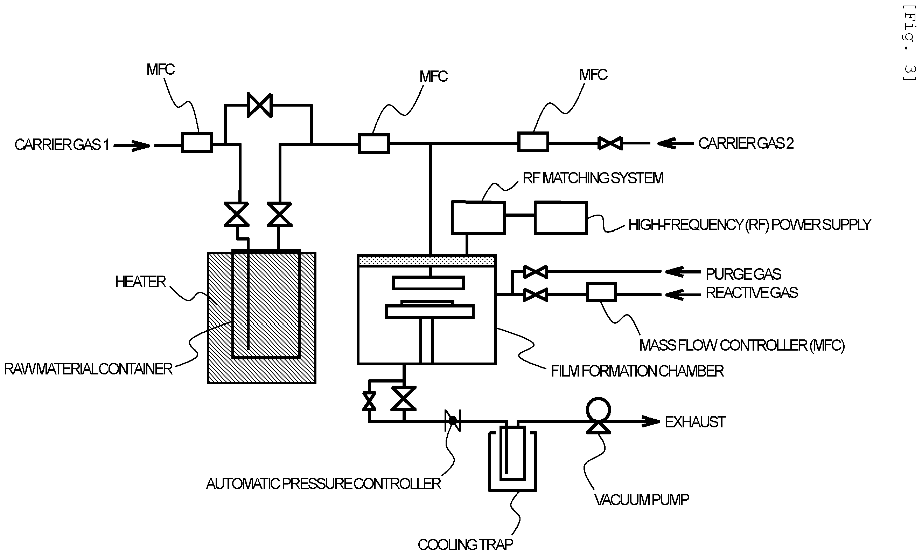

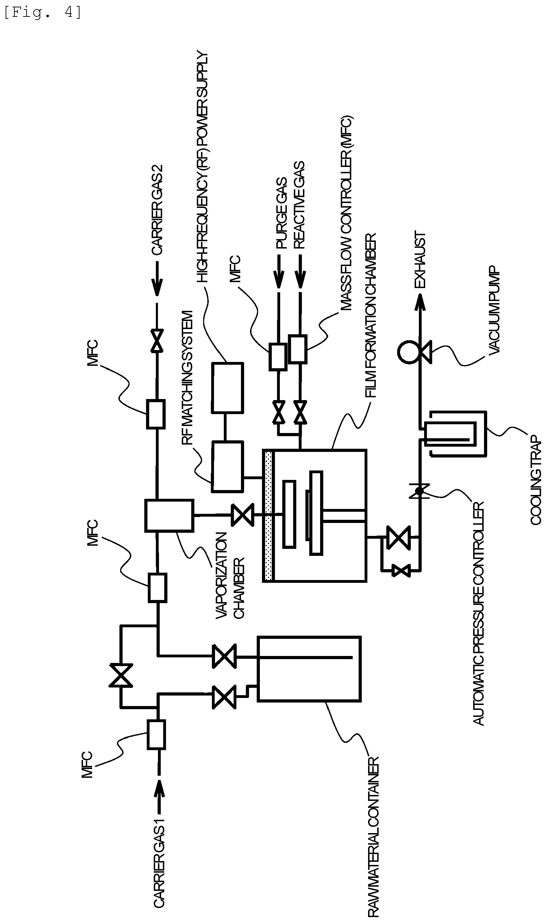

Regarding a device for producing a thin film using the thin-film-forming raw material of the present invention, a well-known chemical vapor deposition device can be used. Specific examples of devices include a device that can perform bubbling supply of a precursor as shown in Fig. 1 and a device having a vaporization chamber as shown in Fig. 2. In addition, a device that can perform a plasma treatment on a reactive gas as shown in Fig. 3 and Fig. 4 may be exemplified. A device that can simultaneously process a plurality of wafers using a batch furnace can be used without limitation to a single-wafer type device as shown in Fig. 1 to Fig. 4.

A thin film produced using the thin-film-forming raw material of the present invention can be made into a thin film of a desired type such as a metal, oxide ceramics, nitride ceramics, and glass by appropriately selecting other precursors, reactive gases and production conditions. It is known that the thin film exhibits electrical characteristics, optical characteristics, and the like, and is applied for various applications. For example, an yttrium-containing thin film is used as an YBCO-based superconductor. In addition, a lanthanum-containing thin film is used as a ferroelectric PLZT. In addition, many lanthanoid atoms are used as dopants for imparting a function of improving electrical characteristics of a specific thin film and a function of improving luminosity.

### Examples

Hereinafter, the present invention will be described in more detail with reference to examples, production examples, comparative examples, and evaluation examples. However, the present invention is not limited to the following examples and the like.

### [Example 1] Compound No. 26

22 g of yttrium-tris-trimethylsilylamide and 86 g of dehydrated toluene were put into a reaction flask and sufficiently mixed. 23 g of 2-ethylimino-3-ethylpentan-3-ol was added dropwise to the suspension at room temperature (20°C). After dropwise addition was completed, the suspension became white, and was changed to a light yellow transparent liquid immediately after heating at 60°C. Then, the solvent was removed in an oil bath at 100°C under a reduced pressure, and then the solid was dried in an oil bath at 113°C under a reduced pressure. The generated yttrium complex (reddish brown viscous liquid) was put into a flask, and distillation purification was performed. Distillation purification was performed at a heating temperature of 189°C and a pressure of 20 Pa, and 9.1 g of a light yellow solid was obtained. The melting point of the obtained solid was 51°C.

### (Analysis value)

(1) Atmospheric pressure TG-DTA
   Mass 50% reduction temperature: 278°C (Ar flow rate: 100 ml/min, temperature increase 10°C/min)
(2) Reduced pressure TG-DTA
   Mass 50% reduction temperature: 194°C (Ar flow rate: 50 ml/min, temperature increase 10°C/min)
(3) 1H-NMR (heavy benzene)
   1.00 ppm (6 H, triplet), 1.30 (3 H, triplet), 1.44 ppm (3 H, singlet), 1.55 to 1.70 ppm (4 H, multiplet), 3.43 ppm (2 H, qualtet)
(4) Elemental analysis (theoretical value)
   C: 58.3% (58.2%), H: 9.9% (9.8%), Y: 15.7% (15.9%), N: 7.3% (7.5%), O: 8.8% (8.6%)

### [Example 2] Compound No. 44

3.4 g of lanthanum-tris-trimethylsilylamide and 21 g of dehydrated toluene were put into a reaction flask and sufficiently mixed. 2.4 g of 2-ethylimino-3-methylpentan-3-ol was added dropwise to the suspension at room temperature (20°C) . The mixture was stirred at room temperature for 24 hours and a light yellow transparent solution was then obtained. Then, the solvent was removed in an oil bath at 110°C under a reduced pressure, and the solid was dried in an oil bath at 120°C under a reduced pressure. The generated lanthanum complex (light yellow liquid) was put into a flask and connected to a small purification device. Distillation purification was performed at a heating temperature of 200°C and 30 Pa and 1.0 g of a light yellow liquid was obtained. The melting point of the obtained solid was 63°C.

### (Analysis value)

(1) Atmospheric pressure TG-DTA
   Mass 50% reduction temperature: 276°C (Ar flow rate: 100 ml/min, temperature increase 10°C/min)
(2) Reduced pressure TG-DTA
   Mass 50% reduction temperature: 250°C (Ar flow rate: 50 ml/min, temperature increase 10°C/min)
(3) 1H-NMR (heavy benzene)
   1.06 ppm (3 H, triplet), 1.19 ppm (3 H, triplet), 1.56 ppm (3 H, broad), 1.64 ppm (3 H, singlet), 1.71 ppm (1 H, multiplet), 1.87 ppm (1 H, multiplet)
(4) Elemental analysis (theoretical value)
   C: 50.8% (51.0%), H: 8.7% (8.6%), La: 24.6% (24.6%), N: 7.1% (7.4%), O: 8.8% (8.5%)

### [Example 3] Compound No. 47

A 14 g of lanthanum-tris-trimethylsilylamide and 86 g of dehydrated toluene were put into a reaction flask and sufficiently mixed. 12 g of 2-ethylimino-3-ethylpentan-3-ol was added dropwise to the suspension at room temperature (20°C). After heating, the mixture was stirred and reacted at room temperature for 20 hours. Then, the solvent was removed in an oil bath at 105°C under a reduced pressure. Then, drying was performed in an oil bath at 125°C under a reduced pressure. The generated lanthanum complex (reddish brown viscous liquid) was put into a flask, and distillation purification was performed. Distillation purification was performed at a heating temperature of 175°C and a pressure of 30 Pa, and 5.2 g of a light yellow solid was obtained. The melting point of the obtained solid was 69°C.

### (Analysis value)

(1) Atmospheric pressure TG-DTA
   Mass 50% reduction temperature: 258°C (Ar flow rate: 100 ml/min, temperature increase 10°C/min)
(2) Reduced pressure TG-DTA
   Mass 50% reduction temperature: 200°C (Ar flow rate: 50 ml/min, temperature increase 10°C/min)
(3) 1H-NMR (heavy benzene)
   1.02 ppm (6 H, triplet), 1.39 (3 H, triplet), 1.45 ppm (3 H, singlet), 1.57 to 1.64 ppm (4 H, multiplet), 3.3 ppm (2 H, qualtet)
(4) Elemental analysis (theoretical value)
   C: 53.6% (53.4%), H: 9.1% (9.0%), La: 22.8% (22.9%), N: 6.9% (6.9%), O: 7.7% (7.9%)

### [Example 4] Compound No. 341

1.0 g of lutetium-tris-trimethylsilylamide and 20 g of dehydrated toluene were put into a reaction flask and sufficiently mixed. 0.72 g of 2-ethylimino-3-ethylpentan-3-ol was added dropwise to the suspension at room temperature (20°C). The white suspension was changed to a light yellow transparent liquid. After dropwise addition was completed, stirring was performed at room temperature overnight. Then, the solvent was removed in an oil bath at 105°C under a reduced pressure and the generated lutetium complex (light yellow viscous liquid) was sufficiently dried. The lutetium complex was put into a flask and connected to a sublimation and purification device. Sublimation and purification were performed at a device heating temperature of 180°C and 30 Pa, and 0.5 g of a white solid was obtained. The melting point of the obtained solid was 185°C.

### (Analysis value)

(1) Atmospheric pressure TG-DTA
   Mass 50% reduction temperature: 277°C (Ar flow rate: 100 ml/min, temperature increase 10°C/min)
(2) Reduced pressure TG-DTA
   Mass 50% reduction temperature: 192°C (Ar flow rate: 50 ml/min, temperature increase 10°C/min)
(3) 1H-NMR (heavy benzene)
   1.00 ppm (6 H, triplet), 1.28 ppm (3 H, triplet), 1.45 ppm (3 H, singlet) , 1.55 to 1.75 ppm (4 H, multiplet), 3.48 ppm (2 H, qualtet)
(4) Elemental analysis (theoretical value)
   C: 50.6% (50.4%), H: 8.3% (8.46%), Lu: 27.0% (27.2%), N: 6.3% (6.53%), O: 7.5% (7.46%)

### [Evaluation Example 1] Evaluation of physical properties of alkoxide compound

The melting point of the metal alkoxide compounds Nos. 26, 44, 47, and 314 of the present inventions obtained in Examples 1 to 4 and the following Comparative Compounds 1, 2 and 3 was measured using a micro melting point measuring device. In addition, using a TG-DTA measuring device, a temperature when the mass of the sample was reduced by 50 mass% due to heating (hereinafter abbreviated as "temperature at TG 50% reduction" in some cases) under a reduced pressure atmosphere (10 torr) was checked. It was possible to determine that a compound having a low melting point was preferable because transportability was favorable, and a compound having a low temperature at TG 50% reduction was preferable because the vapor pressure was high. The results are shown in Table 1, Table 2 and Table 3.

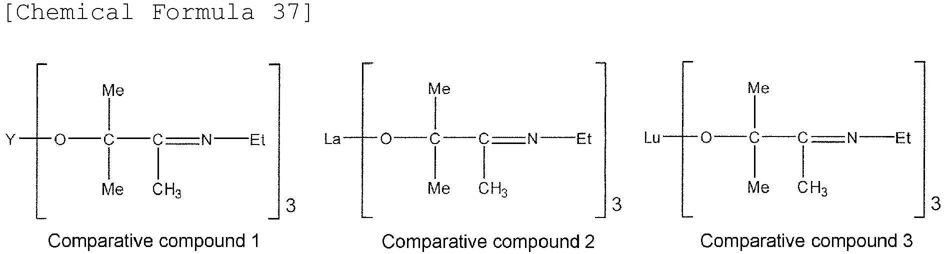

### [Table 1]

**Table 1**

| Evaluation Example | Compound | Melting point (°C) | Temperature at TG 50% reduction (°C) |
|---|---|---|---|
| Evaluation Example 1-1 | Compound No. 26 | 51 | 194 |
| Comparative Example 1 | Comparative Compound 1 | 183 | 261 |

Based on the results in Table 1, comparing Compound No. 26 and Comparative Compound 1 as a tertiary aminoalkoxide yttrium compound having a structure similar thereto, it was found that Compound No. 26 had a low melting point and had a low temperature at TG 50% reduction. It was found that, since Compound No. 26 had a lower melting point than Comparative Compound 1 and also had a high vapor pressure, it was a compound suitable as a thin-film-forming raw material.

### [Table 2]

**Table 2**

| Evaluation Example | Compound | Melting point (°C) | Temperature at TG 50% reduction (°C) |
|---|---|---|---|
| Evaluation Example 1-2 | Compound No. 44 | 63 | 250 |
| Evaluation Example 1-3 | Compound No. 47 | 69 | 197 |
| Comparative Example 2 | Comparative Compound 2 | >100 | 260 |

Based on the results in Table 2, comparing Compound No. 44, Compound No. 47, and Comparative Compound 2 as a tertiary aminoalkoxide lanthanum compound having a structure similar thereto, it was found that Compound No. 44 and Compound No. 47 had a low melting point and a low temperature at TG 50% reduction. Among these, it was found that Compound No. 47 had a particularly low temperature at TG 50% reduction and a high vapor pressure. It was found that, since Compound No. 44 and Compound No. 47 had a lower melting point than Comparative Compound 2 and also had a high vapor pressure, they were compounds suitable as a thin-film-forming raw material.

### [Table 3]

**Table 3**

| Evaluation Example | Compound | Melting point (°C) | Temperature at TG 50% reduction (°C) |
|---|---|---|---|
| Evaluation Example 1-4 | Compound No. 341 | 185 | 260 |
| Comparative Example 3 | Comparative Compound 3 | >230 | - *1 |

| | | | |
|---|---|---|---|
| *1 Even if heated to 600°C, reduction in mass by 50 mass% was not confirmed. | | | |

Based on the results in Table 3, comparing Compound No. 341 and Comparative Compound 3 as a tertiary aminoalkoxide lutetium compound having a structure similar thereto, it was found that Compound No. 341 had a low melting point and a low temperature at TG 50% reduction.

In addition, based on the results of the temperature at TG 50% reduction measured for Comparative Example 3, the mass was reduced only by 40 to 45 mass% even if Comparative Compound 3 was heated to 600°C, and thus it was found that Comparative Compound 3 also had a low vapor pressure and was not suitable as a thin-film-forming raw material. It was found that, since Compound No. 26 had a lower melting point than Comparative Compound 1 and a high vapor pressure, it was a compound suitable as a thin-film-forming raw material.

### [Example 5] Production of yttrium oxide thin film

Compound No. 26 was used as a raw material for an atomic layer deposition method, and according to the ALD method under the following conditions using a device shown in Fig. 1, an yttrium oxide thin film was produced on a silicon wafer.

When the thin film composition of the obtained thin film was checked through X-ray photoelectron spectroscopy, the obtained thin film had a composition of yttrium oxide (Y:O = 2:3), and the carbon content was smaller than 0.1 atom% which was a detection lower limit. In addition, when the film thickness was measured according to an X-ray reflectance method and its average value was calculated, the film thickness on average was 70 nm, and the film thickness obtained for one cycle on average was 0.07 nm. In addition, based on the result of the cross section observed through FE-SEM, the surface of the thin film was smooth.

### (Conditions)

Substrate: silicon wafer
Reaction temperature (silicon wafer temperature): 325°C
Reactive gas: H₂O

A series of processes including the following (1) to (4) was set as one cycle and repeated over 1,000 cycles:
(1) a raw material for an atomic layer deposition method vaporized under conditions of a raw material container temperature of 160°C and a pressure in the raw material container of 100 Pa was introduced into a film-forming chamber and deposition was performed at a system pressure of 100 Pa for 30 seconds;
(2) purging with argon gas was performed for 15 seconds and thereby an un-deposited raw material was removed;
(3) a reactive gas was introduced into the film-forming chamber and reacted at a system pressure of 100 Pa for 0.2 seconds; and
(4) purging with argon gas was performed for 15 seconds and thereby an unreacted first reactive gas and by-product gas were removed.

### [Comparative Example 4] Production of yttrium oxide thin film

Comparative Compound 1 was used as a raw material for an atomic layer deposition method, and an yttrium oxide thin film was produced on a silicon substrate under the same conditions as in Example 5. When the film thickness of the obtained thin film was measured according to the X-ray reflectance method, and the thin film structure and the thin film composition were checked according to an X-ray diffraction method and X-ray photoelectron spectroscopy, the film thickness was 4 nm, the film had a composition of yttrium oxide, and the residual carbon content in the thin film was 10 atom% or more. The film thickness obtained for one cycle was 0.004 nm.

Based on the results of Example 5, it was found that, when Compound No. 26 was used as a thin-film-forming raw material for an ALD method, it was possible to form an yttrium oxide thin film having excellent quality. On the other hand, it was found that the thin film obtained using Comparative Compound 1 as a thin-film-forming raw material for an ALD method was a thin film having a large residual carbon content and poor quality.

### [Example 6] Production of lanthanum oxide thin film

Compound No. 44 was used as a raw material for an atomic layer deposition method, and according to the ALD method under the following conditions using a device shown in Fig. 1, a lanthanum oxide thin film was produced on a silicon wafer.

When the thin film composition of the obtained thin film was checked through the X-ray photoelectron spectroscopy, the obtained thin film had a composition of lanthanum oxide (La:O = 2:3) and the carbon content was smaller than 0.1 atom% which was a detection lower limit. In addition, when the film thickness was measured according to the X-ray reflectance method and its average value was calculated, the film thickness was 60 nm, and the film thickness obtained for one cycle on average was 0.06 nm. In addition, based on the result of the cross section observed through FE-SEM, the surface of the thin film was smooth.

### (Conditions)

Substrate: silicon wafer
Reaction temperature (silicon wafer temperature): 325°C
Reactive gas: H₂O

A series of processes including the following (1) to (4) was set as one cycle and repeated over 1,000 cycles:
(1) a raw material for an atomic layer deposition method vaporized under conditions of a raw material container temperature of 160°C and a pressure in the raw material container of 100 Pa was introduced into a film-forming chamber and deposition was performed at a system pressure of 100 Pa for 30 seconds;
(2) purging with argon gas was performed for 15 seconds and thereby an un-deposited raw material was removed;
(3) a reactive gas was introduced into the film-forming chamber and reacted at a system pressure of 100 Pa for 0.2 seconds; and
(4) purging with argon gas was performed for 15 seconds and thereby an unreacted first reactive gas and by-product gas were removed.

### [Example 7] Production of lanthanum oxide thin film

Compound No. 47 was used as a raw material for an atomic layer deposition method, and a silicon substrate was produced on a lanthanum oxide thin film under the same conditions as in Example 6. When the thin film composition of the obtained thin film was checked through the X-ray photoelectron spectroscopy, the obtained thin film had a composition of lanthanum oxide (La:O = 2:3), and the carbon content was smaller than 0.1 atom% which was a detection lower limit. In addition, when the film thickness was measured according to the X-ray reflectance method and its average value was calculated, the film thickness was 70 nm, and the film thickness obtained for one cycle on average was 0.07 nm. In addition, based on the result of the cross section observed through FE-SEM, the surface of the thin film was smooth.

### [Comparative Example 5] Production of lanthanum oxide thin film

Comparative Compound 2 was used as a raw material for an atomic layer deposition method and a lanthanum oxide thin film was produced on a silicon substrate under the same conditions as in Example 6. When the film thickness of the obtained thin film was measured according to the X-ray reflectance method, and the thin film structure and the thin film composition were checked according to the X-ray diffraction method and the X-ray photoelectron spectroscopy, the film thickness was 3 nm, and the film had a composition of lanthanum oxide, and the residual carbon content in the thin film was 10 atom% or more. The film thickness obtained for one cycle was 0.003 nm.

Based on the results of Example 6 and Example 7, it was found that, when Compound No. 44 and Compound No. 47 were used as a thin-film-forming raw material for an ALD method, it was possible to form a lanthanum oxide thin film having excellent quality. On the other hand, it was found that the thin film obtained using Comparative Compound 2 as a thin-film-forming raw material for an ALD method was a thin film having a large residual carbon content and poor quality.

### [Example 8] Production of lutetium oxide thin film

Compound No. 341 was used as a raw material for an atomic layer deposition method, and according to the ALD method under the following conditions using a device shown in Fig. 1, a lutetium oxide thin film was produced on a silicon wafer.

When the thin film composition of the obtained thin film was checked through the X-ray photoelectron spectroscopy, the obtained thin film had a composition of lutetium oxide (Lu:O = 2:3), and the carbon content was smaller than 0.1 atom% which was a detection lower limit. In addition, when the film thickness was measured according to the X-ray reflectance method and its average value was calculated, the film thickness was 65 nm, and the film thickness obtained for one cycle on average was 0.065 nm. In addition, based on the result of the cross section observed through FE-SEM, the surface of the thin film was smooth.

### (Conditions)

Substrate: silicon wafer
Reaction temperature (silicon wafer temperature): 325°C
Reactive gas: H₂O

A series of processes including the following (1) to (4) was set as one cycle and repeated over 1,000 cycles:
(1) a raw material for an atomic layer deposition method vaporized under conditions of a raw material container temperature of 160°C and a pressure in the raw material container of 100 Pa was introduced into a film-forming chamber and deposition was performed at a system pressure of 100 Pa for 30 seconds;
(2) purging with argon gas was performed for 15 seconds and thereby an un-deposited raw material was removed;
(3) a reactive gas was introduced into the film-forming chamber and reacted at a system pressure of 100 Pa for 0.2 seconds; and
(4) purging with argon gas was performed for 15 seconds and thereby an unreacted first reactive gas and by-product gas were removed.

### [Comparative Example 6] Production of lutetium oxide thin film

Comparative Compound 3 was used as a raw material for an atomic layer deposition method, and a lutetium oxide thin film was produced on a silicon substrate under the same conditions as in Example 6. When the film thickness of the obtained thin film was measured according to the X-ray reflectance method, and the thin film structure and the thin film composition were checked according to the X-ray diffraction method and the X-ray photoelectron spectroscopy, the film thickness was 4 nm, the film had a composition of lutetium oxide, and the residual carbon content in the thin film was 3 atom% or more. The film thickness obtained for one cycle was 0.004 nm.

Based on the results of Example 8, it was found that, when Compound No. 341 was used as a thin-film-forming raw material for an ALD method, it was possible to form a lutetium oxide thin film having excellent quality. On the other hand, it was found that the thin film obtained using Comparative Compound 3 as a thin-film-forming raw material for an ALD method was a thin film having a large residual carbon content and poor quality.

## Claims

1. A metal alkoxide compound represented by the following general formula (1): 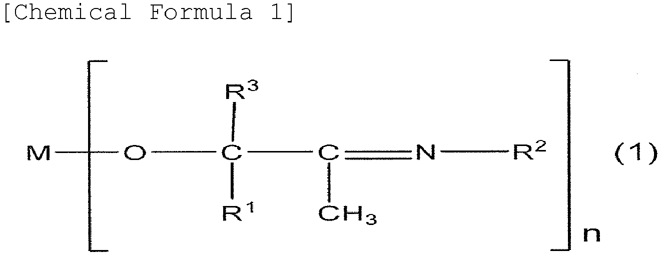 (in the formula, R¹ and R² each independently represent an alkyl group having 1 to 4 carbon atoms, R³ represents an alkyl group having 2 or 3 carbon atoms, M represents a scandium atom, an yttrium atom, a lanthanum atom, a cerium atom, a praseodymium atom, a neodymium atom, a promethium atom, a samarium atom, a europium atom, a gadolinium atom, a terbium atom, a dysprosium atom, a holmium atom, an erbium atom, a thulium atom, an ytterbium atom, or a lutetium atom, and n represents the valence represented by M.)

2. A thin-film-forming raw material including the metal alkoxide compound according to claim 1.

3. A method of producing a thin film containing at least one atom selected from among a scandium atom, an yttrium atom, a lanthanum atom, a cerium atom, a praseodymium atom, a neodymium atom, a promethium atom, a samarium atom, a europium atom, a gadolinium atom, a terbium atom, a dysprosium atom, a holmium atom, an erbium atom, a thulium atom, an ytterbium atom, and a lutetium atom on a surface of a substrate, the method comprising
a process in which vapor containing a metal alkoxide compound obtainable by vaporizing the thin-film-forming raw material including the metal alkoxide compound according to claim 2 is introduced to a processing atmosphere, and the metal alkoxide compound is decomposed and/or chemically reacted to be deposited on the surface of the substrate.
